# EUROPEAN PATENT SPECIFICATION

(11) **EP 3 631 453 B1**
(45) Date of publication and mention of the grant of the patent: **29.10.2025**
(21) Application number: 18731219.4
(22) Date of filing: 23.05.2018
(51) Int. Cl.: G01N 33/574

(54) **NOVEL STOOL-BASED PROTEIN BIOMARKERS FOR COLORECTAL CANCER SCREENING**
NEUARTIGE STUHLBASIERTE PROTEINBIOMARKER FÜR KOLOREKTALKREBS-SCREENING
NOUVEAUX BIOMARQUEURS PROTÉINIQUES BASÉS SUR LES SELLES POUR LE DÉPISTAGE DU CANCER COLORECTAL

(30) Priority: 23.05.2017 EP 17172531
(43) Date of publication of application: 08.04.2020
(73) Proprietor: Stichting Het Nederlands Kanker Instituut- Antoni van Leeuwenhoek Ziekenhuis, 1066 CX Amsterdam (NL); Stichting Amsterdam UMC, 1081 HV Amsterdam (NL)
(72) Inventor: BOSCH, Linda Janna Willemien, 1066 CX Amsterdam (NL); DE WIT, Meike, 1066 CX Amsterdam (NL); PINTO MORAIS DE CARVALHO, Beatriz, 1066 CX Amsterdam (NL); FIJNEMAN, Remondus Johannes Adriaan, 1066 CX Amsterdam (NL); JIMENEZ, Cornelia Ramona, 1081 HV Amsterdam (NL); MEIJER, Gerrit Albert, 1066 CX Amsterdam (NL); COUPÉ, Veerle Marleen Herman, 1081 HV Amsterdam (NL)
(74) Representative: V.O.
(86) International application number: PCT/NL2018/050341
(87) International publication number: WO 2018/217087

(56) References cited:
- WO-A1-2013/162368
- YANYU CHEN ET AL: "Protein content and functional characteristics of serum-purified exosomes from patients with colorectal cancer revealed by quantitative proteomics : Serum-purified exosomes from patients with CRC", INTERNATIONAL JOURNAL OF CANCER, vol. 140, no. 4, 15 February 2017 (2017-02-15), US, pages 900 - 913, XP055489667, ISSN: 0020-7136, DOI: 10.1002/ijc.30496

## Description

### 1. FIELD OF THE INVENTION

The invention relates to the field of oncology. More specifically, the invention relates to methods for typing colorectal cancerous cells. The invention provides methods and means for differentiating colorectal cancerous cells from normal cells, based on biomarkers in stool.

### 2. BACKGROUND OF THE INVENTION

Screening aims to lower the burden of colorectal cancer (CRC) by either preventing cancer development or detecting disease at a curable stage (Segnan et al., 2010. European guidelines for quality assurance in colorectal cancer screening and diagnosis First Edition: European Commission). Although colonoscopy remains the gold standard for detecting colorectal tumors, for reasons of compliance and costs most population-wide screening programs use non-invasive stool-based tests for triage for colonoscopy (Cunningham et al., 2010. Lancet 375: 1030-47). The guaiac-based fecal occult blood test (gFOBT) has proven to reduce mortality from CRC (Mandel et al., 1993. N Engl J Med 328: 1365-71; Hardcastle et al., 1996. Lancet 348: 1472-7; Kewenter et al., 1994. Scand J Gastroenterol 29: 468-73). The more recent fecal immunochemical test (FIT), which uses an antibody against human hemoglobin, outperforms gFOBT and is now widely used (van Rossum et al., 2008. Gastroenterology 135: 82-90; Benson et al., 2012. Int J Cancer 130: 2961-73).

Molecular screening tests have the potential to improve detection of colorectal tumors over FIT (Bosch et al., 2011. Clin Colorectal Cancer 10: 8-23; Imperiale et al., 2014. N Engl J Med 370:1287-1297; Ahlquist, 2015. Dig Dis Sci 60: 623-33). Protein biomarkers can be translated into simple and cost-effective antibody-based screening tests. Ideally, these biomarkers could also be quantified in small stool sample volumes, that are used in FIT-based screening programs. However, thus far, alternative protein biomarkers have failed to improve current hemoglobin-based CRC stool screening tests (Bosch et al., 2011. Clin Colorectal Cancer 10: 8-23).

Technological advancements in mass spectrometry now allow for in-depth proteomics for biomarker discovery in complex biological samples (Jimenez and Verheul, 2014. Am Soc Clin Oncol Educ Book:e504-10; Taguchi and Hanash, 2013. Clin Chem 59: 119-26). A classical approach is to first identify discriminating markers in tissue and/or cell line material, followed by validation in the final analyte, such as stool. However, constituents of the analyte ultimately used for screening, in case of stool e.g. bacterial proteases and glycosidases, may affect test performance, possibly leading to validation failure(de Wit et al., 2013. Clin Biochem 46: 466-79; Bosch et al., 2011. Clin Colorectal Cancer 10: 8-23). Therefore, biomarker discovery directly in the biological sample taken for screening, i.e. stool, may be a powerful alternative.

Fecal immunochemical tests (FIT), preferred in recent guidelines, provide an advanced fecal occult blood test (FOBT) technology that uses antibodies against hemoglobin to detect traces of blood in a stool sample. FIT can be adapted for automated test reading and to report quantitative results. However, FIT has sub-optimal sensitivity for CRC (79%) and is even less sensitive for advanced colonic adenomas (AAs), i.e. high-risk precursor lesions (31%) (de Wijkerslooth et al., 2012. Am J Gastroenterol 107: 1570-8; Lee et al., 2014. Ann Intern Med 160:171).

Therefore, there is a need to identify proteins in stool that outperform or complement fecal hemoglobin as a biomarker for early detection of CRC and AA. WO 2013/162368 A1 discloses SERPINF2, S100A8 and HBA1 in several lists of biomarkers, and also discloses methods for typing a stool sample of an individual suffering from colorectal cancer.

### 3. BRIEF DESCRIPTION OF THE INVENTION

The invention provides a method for typing a sample of an individual suffering from a colorectal cancerous growth, or suspected of suffering therefrom, the method comprising
a. providing a stool sample comprising protein expression molecules from cancerous cells or suspected to comprise protein expression molecules from cancerous cells;
b. determining expression levels for said expression molecules of a set of genes in said sample; and
c. typing said sample on the basis of the expression levels determined for said expression molecules of the set of genes; wherein said set of genes consists of SERPINF2, S100A8 and HBA1.

Disclosed but not claimed are sets comprising SERPINF2 and S100A8, more preferably SERPINF2, S100A8 and HBA1, more preferably SERPINF2, S100A8, HBA1 and S100A9, more preferably SERPINF2, S100A8, HBA1, S100A9 and C3. S100A8 and S100A9 are often expressed as a heterodimer, termed S100A8/A9 or calprotectin (CPT). Protein expression molecules from a set of genes comprising at least SERPINF2 and S100A8, preferably SERPINF2, S100A8 and HBA1, are suited for typing a sample of an individual suffering from early adenoma, advanced adenoma and/or colorectal cancer, specifically from advanced adenoma and/or colorectal cancer, or suspected of suffering therefrom.

A preferred sample is or comprises stool. A stool sample can be collected by an individual and sent to a laboratory for analysis. A stool-based test that is currently used to screen for colorectal cancer is a fecal immunochemical test (FIT). A standard FIT test applies antibodies to detect human hemoglobin protein. A positive result will need to be followed by a colonoscopy. However, a cause of hemoglobin in stool is often a non-cancerous condition, such as an ulcer or hemorrhoid.

Important features of a test that is used for population screening are the number of false negatives and false positives, which features are related to sensitivity and specificity of the test. A reduction in the number of false negatives is often associated with an increase in the number of false-positive results. To improve the overall accuracy of a stool-based test, several marker classes including DNA, protein, messenger RNA (mRNA), and micro RNA (miRNA), have been investigated. In fact, a multi-target stool DNA (MT-sDNA) test has been recently shown to have superior sensitivity, although with lower specificity, to fecal hemoglobin by immunochemical testing for the detection of curable-stage CRC and advanced adenomas and to have an overall cancer detection similar to colonoscopy (Imperiale et al., 2014. N Engl J Med 370:1287-1297).

A method according to the present invention is safe and simple to use, provides high sensitivity for early stage colorectal cancer, including detection of advanced adenoma's, provides detection of a cancerous growth throughout the bowel, and combines high specificity with high sensitivity.

Disclosed but not claimed are sets comprising three or more genes selected from SERPINF2, S100A8, S100A9, C3, CP, TF, CAT, C9, LTF, HBB, HPX, HBA1, HP, GPI, MPO, HBD, A2M, CDA, FGG, AZU1, VTN, RBP4, KNG1, PSMA5, C5, FN1, LDHA, PRTN3 and GSR, more preferred comprises or consists of SERPINF2, S100A8, C3, HPX, HBA1, HP, MPO, A2M, RBP4 and LTF.

A level of expression of a protein expression molecule selected from SERPINF2, S100A8, S100A9, C3, CP, TF, CAT, C9, LTF, HBB, HPX, HBA1, HP, GPI, MPO, HBD, A2M, CDA, FGG, AZU1, VTN, RBP4, KNG1, PSMA5, C5, FN1, LDHA, PRTN3 and GSR is preferably determined with an antibody or a functional part thereof directed against said protein expression molecule.

With the term typing, as is used in a method of the invention, is meant assessing presence and/or staging of said colorectal cancerous growth, preferably differentiating advanced adenoma and colorectal cancer from non-cancerous growth, including normal colorectal tissue, more preferably differentiating colorectal cancer cells from other cell types.

A preferred method comprises the steps of (a) providing a stool sample from an individual; (b) extracting protein expression molecules from said stool sample; (c) reacting said extracted protein expression molecules with at least two different antibodies, directed against at least two extracted protein expression molecules, whereby said at least two protein expression molecules are expression molecules of SERPINF2, S100A8, S100A9, C3, CP, TF, CAT, C9, LTF, HBB, HPX, HBA1, HP, GPI, MPO, HBD, A2M, CDA, FGG, AZU1, VTN, RBP4, KNG1, PSMA5, C5, FN1, LDHA, PRTN3 and/or GSR; (d) quantifying reaction products between said at least two antibodies and said at least two extracted protein expression molecules; and (e) determining a level of expression of said at least two extracted protein expression molecules, based on the quantified reaction products.

The invention further provides a kit consisting of reagents for directly or indirectly determining a level of expression of a set of proteins consisting of SERPINF2, S100A8 and HBA1 in a stool sample and preferably reagents for an immunochemical assay.

Alternative kits which are not claimed comprise two antibodies directed against protein expression products of at least two genes selected from SERPINF2, S100A8, S100A9, C3, CP, TF, CAT, C9, LTF, HBB, HPX, HBA1, HP, GPI, MPO, HBD, A2M, CDA, FGG, AZU1, VTN, RBP4, KNG1, PSMA5, C5, FN1, LDHA, PRTN3 and GSR, preferably selected from the group consisting of SERPINF2, S100A8, S100A9, C3, CP, TF, CAT, C9, LTF, HBB, HPX, HBA1, HP, GPI, MPO, HBD, A2M, CDA, FGG, AZU1, VTN, RBP4, KNG1, PSMA5, C5, FN1, LDHA, PRTN3 and GSR.

Said at least two different antibodies are preferably arranged in an arrayed format. An advantage of an arrayed format is that it allows image-based screening. Further, the arrayed format enables automation of the assay using standard, multi-well robotics, greatly accelerating the process and reducing cost, allowing high throughput screening.

Also disclosed are methods comprising (f) comparing said determined expression levels with the expression levels of said at least two extracted protein expression molecules in a reference; (g) determining a similarity value between a level of expression of said at least two extracted protein expression molecules in said individual and a level of expression of the at least two extracted protein expression molecules in a patient not having a cancerous growth; and (h) classifying said individual as having a cancerous growth if said similarity value is below a first similarity threshold value, and classifying said individual as not having a cancerous growth if said similarity value exceeds said first similarity threshold value.

The specification further provides a method of assigning treatment to an individual suffering from colorectal cancer, said method comprising (a) classifying said individual as having a cancerous growth or as not having a cancerous growth according to the invention; (b) assigning treatment comprising colonoscopy if said individual is classified as having said cancerous growth.

A preferred method of assigning treatment further comprises removing at least part of the cancerous growth if presence of a cancerous growth is confirmed by colonoscopy.

A preferred method of assigning treatment further comprises assigning 5-fluoruracil (5-FU), preferably further in combination with leucovorin.

A preferred method of assigning treatment further comprises assigning 5-FU, leucovorin and oxaliplatin or 5-FU, leucovorin and irinotecan.

### 4. FIGURE LEGENDS

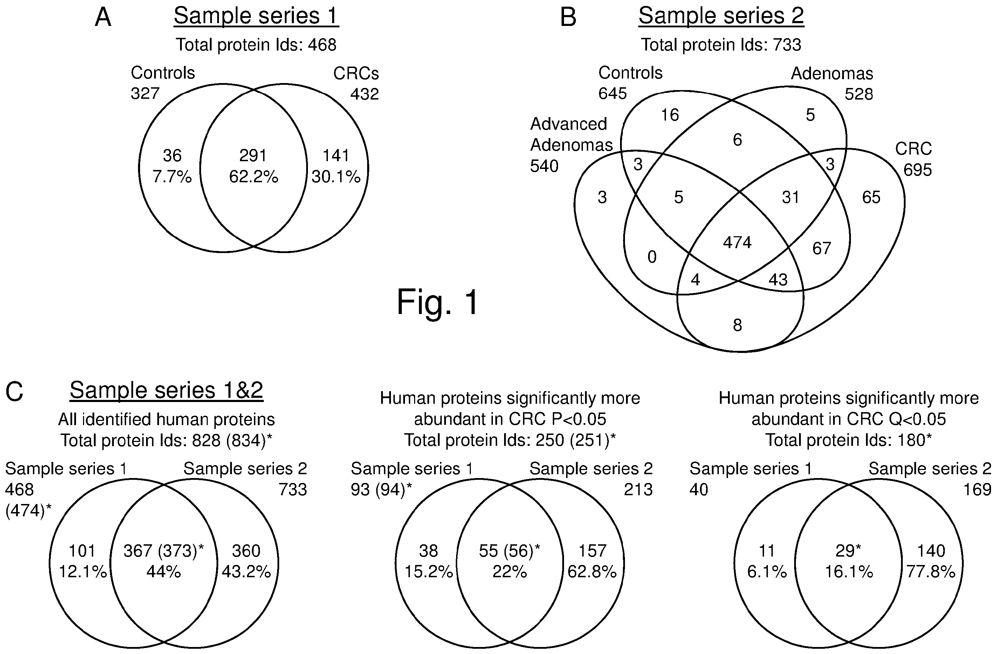

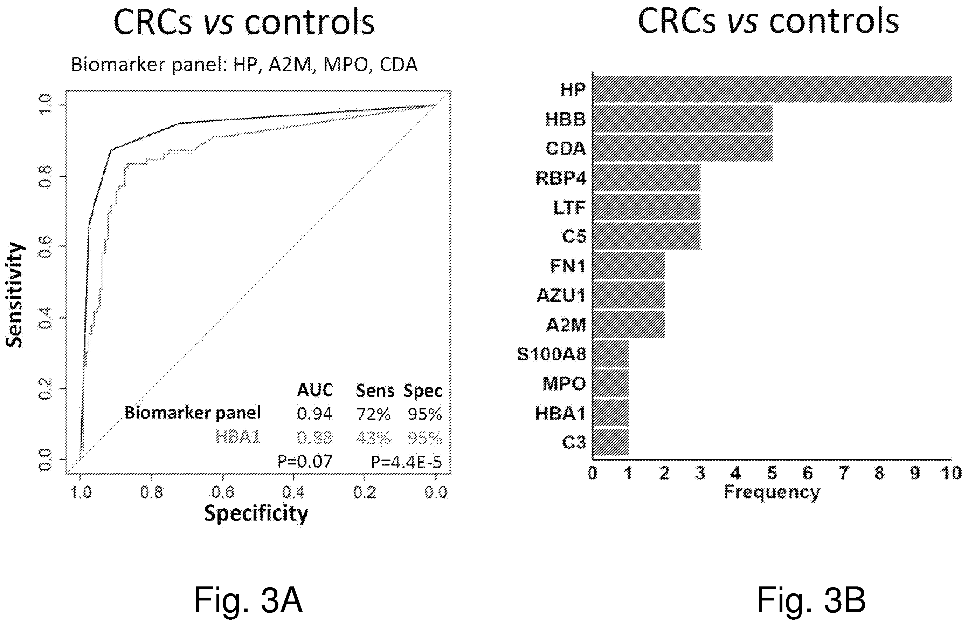

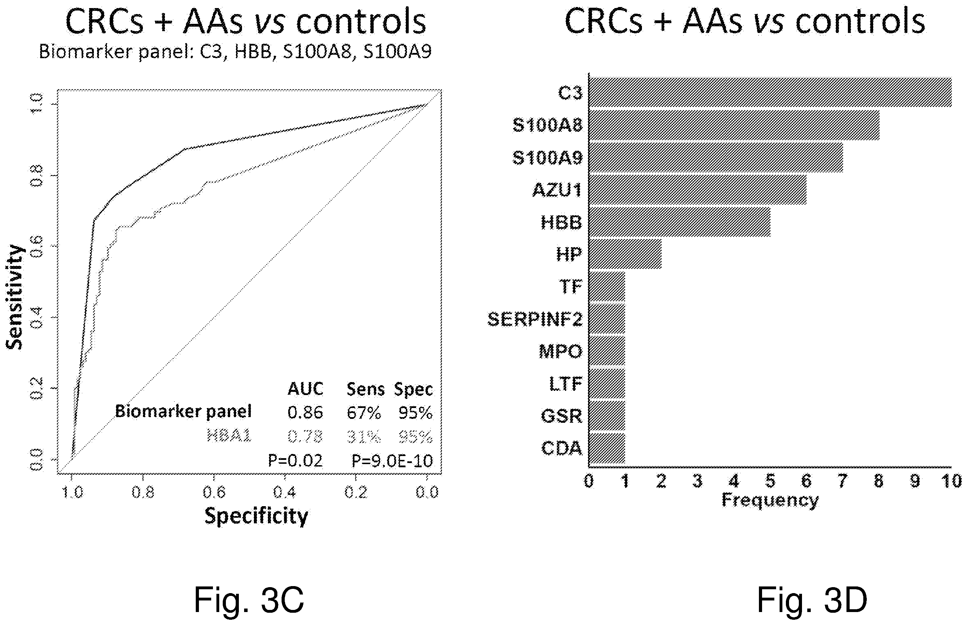

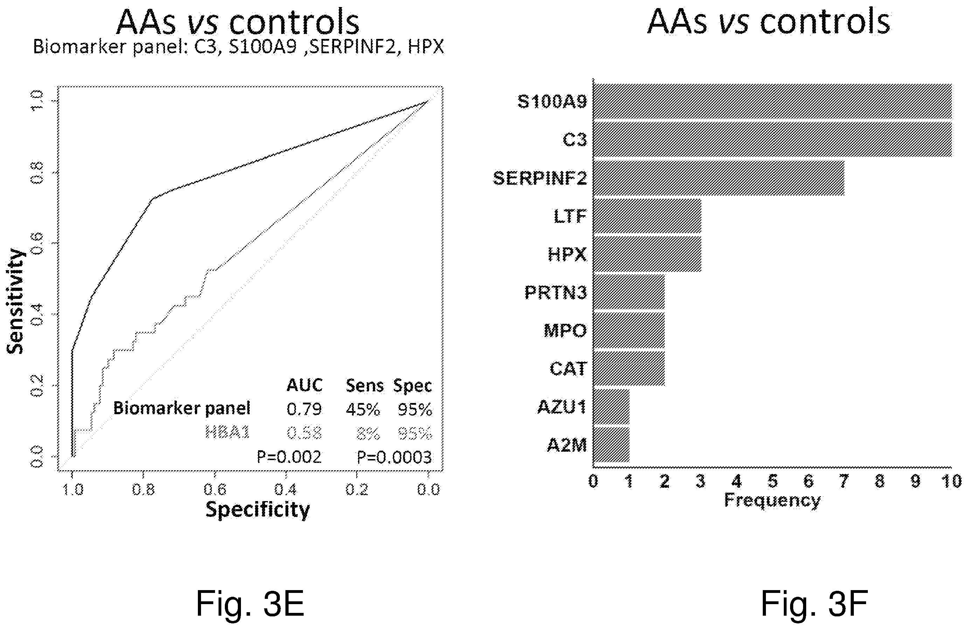

Figure 1. Number of proteins identified in stool samples series 1 and 2.
   Shown are venn diagrams indicating the number of human proteins identified in stool samples from different categories and their overlap in sample series 1 (A) and sample series 2 (B). The different categories include stool samples from control individuals, adenoma patients, advanced adenoma patients, and colorectal cancer (CRC) patients. In (C), venn diagrams of the overlap of the total number of identified human proteins in sample series 1 and sample series 2 (left figure), the number of significantly more abundant proteins in CRCs versus controls (p<0.05) (middle figure) and the number of significantly more abundant proteins in CRCs versus controls with Q<0.05 (right figure). In all venn diagrams, numbers are presented as absolute number of proteins and as percentages from the total number of identified proteins.
   * some proteins were detected in two isoforms in sample series 2, while a single isoform was detected in sample series 1. Therefore, for a fair comparison in the overlap with dataset 2, the protein ids in dataset 1 should be adapted to the numbers in between brackets. No isoforms were present in the list of human proteins significantly more abundant proteins in CRCs versus controls, hence these numbers remain the same.

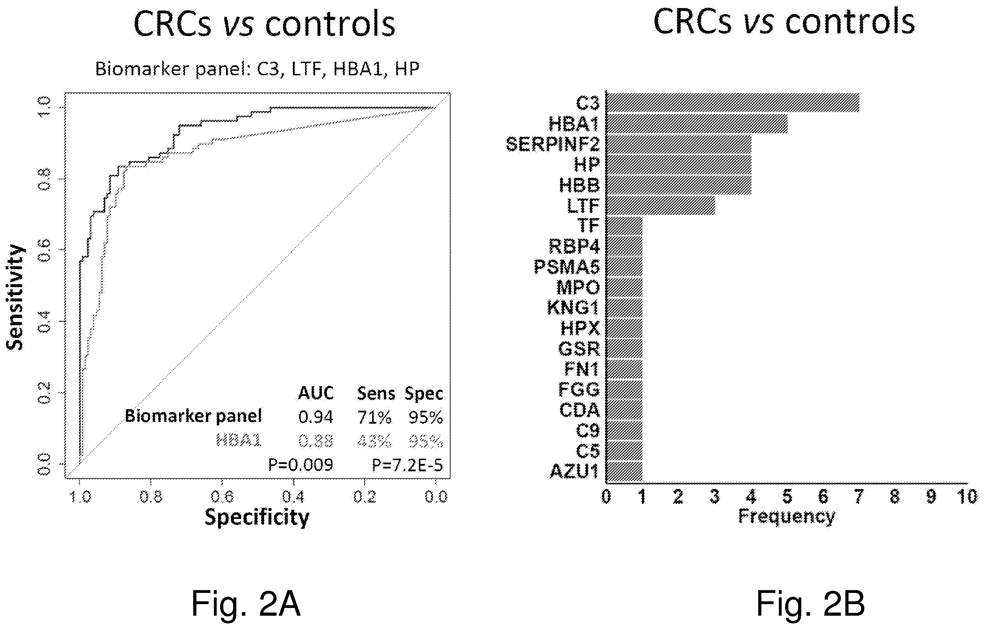

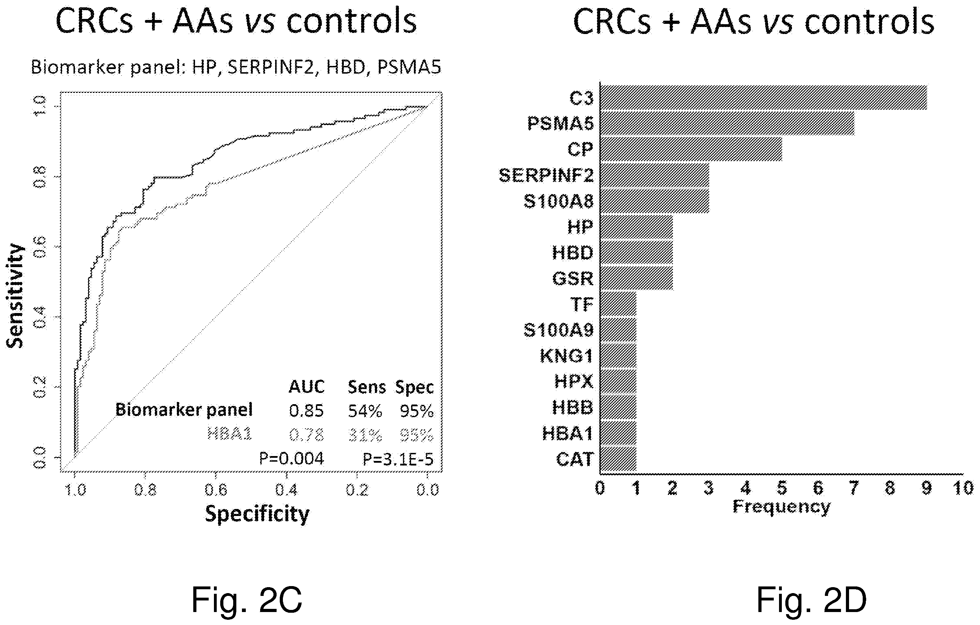

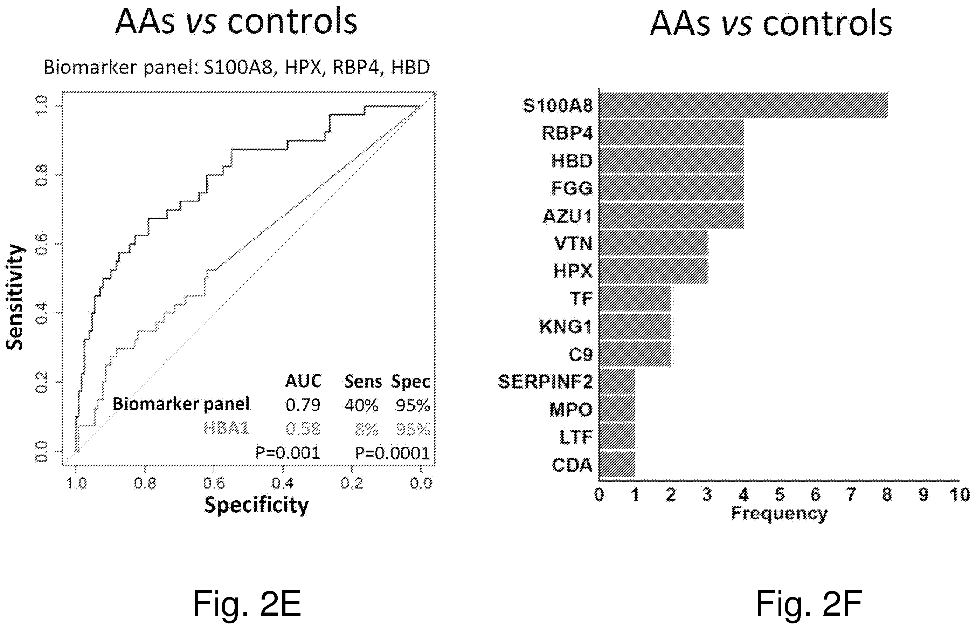

Figure 2. Biomarker panels from logistic regression analysis on sample series 2.
   Shown are the Receiver Operating Characteristic (ROC) curves of the best performing biomarker panels versus HBA1 and the frequencies of the individual proteins included in the top-10 best performing biomarker panels for discriminating CRCs from controls (A and B), for discriminating CRCs plus AAs from controls (C and D) and for discriminating AAs from controls (E and F).
   The black and grey lines in the ROC curves show the performance of the biomarker panels and HBA1, respectively. For each biomarker panel and HBA1 performance, the Area under the Curve (AUC) and sensitivity (sens) at 95% specificity (spec) and corresponding P-values are depicted in A, C and E
Figure 3. Biomarker panels from CART analysis on sample series 2.
   Shown are the Receiver Operating Characteristic (ROC) curves of the best performing biomarker panels versus HBA1 and the frequencies of the individual proteins included in the top-10 best performing biomarker panels for discriminating CRCs from controls (A and B), for discriminating CRCs plus AAs from controls (C and D) and for discriminating AAs from controls (E and F).
   The black and grey lines in the ROC curves show the performance of the biomarker panels and HBA1, respectively. For each biomarker panel and HBA1 performance, the Area under the Curve (AUC) and sensitivity (sens) at 95% specificity (spec) and corresponding P-values are depicted in A, C and E.
Figure 4. Biomarker detection in FIT fluids from sample series 3.
   Shown are boxplots of protein levels of A2M (A), MPO (B), RBP4 (C) and adiponectin (D) as measured by an antibody-based assay on FIT samples from controls, adenomas, AAs and CRCs.
Figure 5. ROC curves of selected markers and FIT for a combination of AA and CRC patients *versus* controls.

### 5. DETAILED DESCRIPTION

### 5.1 Definitions

The term cancerous growth, as is used herein, refers to a carcinoma, a cancer of epithelial tissue that covers or lines surfaces of colorectal tract. Said carcinoma preferably is an adenocarcinoma. The term cancerous growth includes early adenoma, advanced adenoma and colorectal cancer.

The term typing, as is used herein, refers to assessing presence and/or staging of said colorectal cancerous growth. The term typing preferably refers to differentiating adenoma's, including early adenoma and advanced adenoma, and colorectal cancer from non-cancerous growth, including normal colorectal tissue. Said typing is intended to provide information to aid in clinical evaluation of colorectal cancer patients. The methods of the invention find particular use in choosing appropriate treatment for said patients.

The term protein expression molecules, as is used herein, refers to protein products of genes.

The term "directly conjugated with a detectable label", as used herein, refers to the labeling of the antibody itself with a detectable label.

The term "indirectly conjugated with a detectable label", as used herein, refers to the indirect labeling of an antibody, for example using a biotin-labelled antibody and a detectable label that is bound to streptavidin, or by using a further antibody that is directed against the indirectly labeled antibody and which further antibody is labeled with a detectable label.

A "detectable label" is a label which may be detected and of which the absolute or relative amount and/or location (for example, the location on an array) can be determined.

The term reference, as is used herein, refers to a sample, preferably a stool sample, that comprises protein expression molecules, preferably proteins, from a healthy individual not suffering from a colorectal cancerous growth or from an individual that is known to suffer from a colorectal cancerous growth. The levels of expression of the protein expression molecules preferably are stored on a computer, or on computer-readable media, to be used in comparisons to the level of expression level data from the sample of the individual.

The term specifically binding, as is used herein, refers to a binding reaction between an antibody-antigen, or other binding pair, which is determinative of the presence of a protein comprising the antigen in a heterogeneous population of proteins and/or other biologics. Thus, under designated conditions, a specified antibody or functional part thereof binds to a particular antigen and does not bind in a significant amount to other proteins present in the sample.

### 5.2 Sample preparation

A sample from an individual suffering from a colorectal cancerous growth, or suspected to suffer therefrom, comprising protein expression molecules can be obtained in numerous ways, as is known to a skilled person, such as by esophagogastroduodenoscopy, colonoscopy, or sigmoidoscopy. Said sample preferably is or comprises stool from an individual suffering from a colorectal cancerous growth, or suspected to suffer from said cancerous growth. A preferred sample is a sample that is obtained from stool by contacting a stool surface, for example with a stick or a brush, and providing a part of the obtained sample in a test tube or on an absorbent surface, for example a test card. Said test tube preferably comprises a buffer, for example a stool stabilization buffer such as a buffer comprising phosphate-buffered saline and sodium azide. A sample comprising protein expression molecules can be freshly prepared at the moment of isolation of the specimen, or it can be prepared from specimen that have been stored, for example at -20°C, until processing for sample preparation.

Alternatively, said specimen can be stored under conditions that preserve the quality of the protein expression products. Examples of these preservative conditions are fixation, addition of protease inhibitors, addition of reducing agents such as dithiothreitol (DTT) or 2-mercaptoethanol (2-ME), and non-aqueous solutions such as Universal Molecular Fixative (Sakura Finetek USA Inc.; US7138226).

A stool sample may be mixed with stool stabilization buffer (Exact Sciences, Madison, WI, USA) immediately after defecation, and processed to a final stool:buffer w/v ratio of between 1:1 and 1:2, preferably between 1.2 and 1.7, more preferably about 1:4, within 72 hours, and stored at - 80°C until use.

Said sample preferably is pretreated to remove contaminants and/or to increase the concentration of the protein expression molecules. This will result in a lower detection limit and will improve reliability of the methods of the invention.

A preferred pretreatment method comprises homogenization in a buffer, for example by vortexing, followed by centrifugation, for example for 15 minutes at 16.000 G. After this, the supernatant may be centrifuged for 10 minutes at full speed. Supernatants may be filtered, for example through a 0.22 µM PVDF filter (Merck Millipore, Billerica, MA, USA) and concentrated using a molecular size cut-off filter, for example a 3 kDa cut-off filter (Amicon Ultra, Merck Millipore, Billerica, MA, USA).

### 5.3 Detection assay

An expression level for a protein expression molecule may determined by any assay known to a skilled person. A level of expression may be determined by polyacrylamide gel electrophoresis, including two dimensional gel electrophoresis, multidimensional protein identification technology, ELISA, bead-based immunoassays, immuno-PCR using, for example, Thunder-Link^{®} antibody-oligonucleotide conjugation kit (Innova Biosciences. Cambridge UK), surface plasmon resonance, liquid chromatography- tandem mass spectrometry (LC-MS/MS), and matrix-assisted laser desorption/ionization time-of-flight mass spectrometry (MALDI-TOF).

Examples are chemo-luminescence assays, fluorescence assays, mass spectrometry, affinity chromatography, Western blotting, Northern blotting, histology and protein expression chips, probes. Preferred are multiplex systems that can measure protein expression molecules from different genes at the same time.

Mass spectrometry is a suitable means of determining a level of expression of a protein. A preferred method comprises liquid chromatography coupled to tandem mass spectrometry in positive electrospray ionization mode. The LC-MS/MS analysis may be performed, for example by using an I-Class UPLC system connected to a Xevo TQS mass spectrometer Waters (Manchester, UK), or an Q Exactive mass spectrometer (Thermo Fisher). A suitable multiplex system for determining an expression level of a protein product is multiple reaction monitoring (MRM), which is a quantitative MS-based approach.

Said protein expression molecules are preferably detected and quantified using an immunochemical assay, preferably employing antibodies, or functional parts thereof, that specifically bind to a ligand on said protein expression molecules, preferably proteins. A protein expression molecule is an antigen for an antibody that specifically reacts with said protein expression molecule. The term antibody, as used herein, refers to an immunoglobulin protein comprising at least a heavy chain variable region (VH), paired with a light chain variable region (VL), that is specific for a target epitope that is present in a protein expression molecule. The term antibody includes synthetic antibody-like molecules or antibody mimics that are known to those skilled in the art such as APTAMERS (Que-Gewirth and Sullenger, 2007. Gene Therapy 14, 283-291); AFFIBODY^{®} molecules (Nord et al., 1995. Prot Eng 8: 601-608), ANTICALINS^{®} (Skerra, 2008. FEBS J. 275: 2677-2683), and AVIMERS^{®} (Silverman et al., 2005. Nat Biotechnol 23: 1556-1561).

The term "functional part of an antibody" is defined herein as a part that has at least one shared property as said antibody in kind, not necessarily in amount. Non-limiting examples of a functional part of an antibody are a single domain antibody, a single chain antibody, a nanobody, an unibody, a single chain variable fragment (scFv), a Fd fragment, a Fab fragment and a F(ab')2 fragment.

The antibodies, or functional parts thereof, are preferably coupled to a solid support such as a bead, monolithic material or a multi-well array. The antibodies, or functional parts thereof, may be coupled directly, or indirectly, for example by coupling of a second antibody that specifically recognizes the antibody that binds to a protein expression molecule. Indirect coupling may be accomplished, for example, by coupling of protein A, protein G, or a mixture of protein A and G to the beads, monolithic material or array. Direct coupling may be accomplished, for example, by cross-linking, covalently binding or physically adsorbing said antibody or part thereof to the solid support.

A preferred method for determining a level of expression of a protein or multiple proteins includes Enzyme-Linked Immuno Sorbent Assay (ELISA) and Flow Cytometric ImmunoAssay (FCIA).

The term "enzyme-linked immunosorbent assay (ELISA)", as is used herein, refers to a plate-based assay that is designed for detecting and quantifying antigens such as protein expression molecules. Said term includes competition ELISA and sandwich ELISA.

In a competition ELISA, known amounts of an antigen are immobilized to a surface. A sample comprising unknown amounts of said antigen is added, and the antigen is subsequently complexed with an antibody that is preferably conjugated, directly or indirectly, to a detectable label such as a colorimetric label, a fluorescent label, a radioactive label or a chemiluminescent label, or an enzyme. Following washing, detection of antibody that is complexed to the immobilized antigen is accomplished by assessing the conjugated label or enzyme activity via incubation with a substrate to produce a measureable product. The amount of label or enzyme activity is inversely proportional to the amount of antigen in the sample.

A preferred assay is a sandwich ELISA, in which a receptacle is coated with a first antibody specific to a protein expression molecule, termed "capture antibody", and detection of bound protein expression molecule is accomplished with a second antibody, termed "detection antibody". It is preferred that the capture and detection antibodies do not interfere with each other and can bind simultaneously to said protein expression molecule.

Said coating of a receptacle or bead, preferably the surface of a receptacle or bead, may be performed directly or indirectly. Indirect coating may be accomplished, for example, by using a biotin-labeled capture antibody that is attached to a linker molecule, for example a U-PLEX Linker (Meso-Scale Discovery, Rockville, USA). The employment of different linker molecules for different capture antibodies allows the generation of arrayed spots on a receptacle, each of which will bind to a specific protein expression molecule. Said receptacle preferably is a multi-well plate, such as a 24 well plate, a 96 well plate or a 384 well plate, in which each of the wells comprises arrayed spots, whereby each of the spots will bind to a specific protein expression molecule.

Said second antibody is preferably directly or indirectly conjugated to a detectable label such as a colorimetric label, a fluorescent label a radioactive label, or a chemiluminescent label, or an enzyme. Detection of the amount of enzyme-conjugated antibody is preferably performed by incubation with a substrate to produce a measureable product. As an alternative, turbidimetric assays are preferred, especially for competition ELISAs.

Detectable labels are well known in the art. A detectable label may be a fluorescent, luminescent, chemiluminescent and/or electrochemiluminescent moiety which, when exposed to specific conditions, may be detected. For example, a fluorescent label may be exposed to radiation (i.e. light) at a specific wavelength and intensity to cause excitation of the fluorescent label, thereby enabling it to emit detectable fluorescence at a specific wavelength that may be detected.

Alternatively, the detectable label may be an enzyme which is capable of converting a (preferably undetectable) substrate into a detectable product that can be visualized and/or detected. Suitable enzymes include horseradish peroxidase, phosphatase, phosphatase/pyrophosphatase and luciferase.

Alternatively, the detectable label may be a radioactive label, which may be incorporated by methods known in the art.

Indirect labeling of an antibody may be accomplished, for example, through conjugation of an antibody or part thereof with biotin and reacting biotin with labelled or enzyme-linked avidin or streptavidin.

As an alternative, carbon coated wells may be equipped with electrodes that produce chemical energy when subjected to an electrical charge, such as the Multi-array^{®} and Multi-spot^{®} 96-well plates of Meso-Scale Discovery. When combined with a SULFO-TAG^{®} antibody, the chemical energy is transformed to emitted light which is measured using a high-resolution CCD camera.

### 5.4 Reference

A level of expression of protein expression molecules is preferably compared with a level of said molecules in a reference. Said reference preferably comprises a stool sample from an individual that is known to suffer from a colorectal cancerous growth, and/or known not to suffer therefrom.

Based on a comparison with the level of expression of the at least two protein expression molecules, preferably proteins, in the reference, it can be determined whether an individual is likely to suffer from a colorectal cancerous growth. For example, when the reference is sample of a person that is known not to suffer from a colorectal cancerous growth, a difference between the determined level of expression of the at least two protein expression molecules, preferably proteins, might indicate that the individual is suffering from a colorectal cancerous growth.

Typing of a sample can be performed in various ways. In one method, a coefficient is determined that is a measure of a similarity or dissimilarity of a sample with said reference. A number of different coefficients can be used for determining a correlation between the determined expression levels in a sample from an individual and the comparative levels of expression in said reference. Preferred methods are parametric methods which assume a normal distribution of the data.

### 5.5 Markers

Disclosed but not claimed is a set of protein expression molecules comprising two or more expression products from SERPINF2, S100A8, S100A9, C3, CP, TF, CAT, C9, LTF, HBB, HPX, HBA1, HP, GPI, MPO, HBD, A2M, CDA, FGG, AZU1, VTN, RBP4, KNG1, PSMA5, C5, FN1, LDHA, PRTN3 and GSR, preferably two or more expression products from SERPINF2, S100A8, C3, LTF, MPO, HBA1, HP, HPX, A2M, and RBP4. An increased level of expression of any one of these markers, when compared to a level of expression in a stool sample of an individual that is known not to suffer from a colorectal cancerous growth, is indicative of the presence of a colorectal cancerous growth.

Said two or more expression products preferably comprise A2M and C3, A2M and LTF, A2M and MPO, A2M and HBA1, A2M and HP, A2M and S100A8, A2M and HPX, A2M and SERPINF2, A2M and RBP4, C3 and LTF, C3 and MPO, C3 and HBA1, C3 and HP, C3 and S100A8, C3 and HPX, C3 and SERPINF2, C3 and RBP4, LTF and MPO, LTF and HBA1, LTF and HP, LTF and S100A8, LTF and HPX, LTF and SERPINF2, LTF and RBP4, MPO and HBA1, MPO and HP, MPO and S100A8, MPO and HPX, MPO and SERPINF2, MPO and RBP4, HBA1 and HP, HBA1 and S100A8, HBA1 and HPX, HBA1 and SERPINF2, HBA1 and RBP4, HP and S100A8, HP and HPX, HP and SERPINF2, HP and RBP4, S100A8 and HPX, S 100A8 and SERPINF2, S100A8 and RBP4, HPX and SERPINF2, HPX and RBP4, SERPINF2 and RBP4, A2M, C3 and LTF, A2M, C3 and MPO, A2M, C3 and HBA1, A2M, C3 and HP, A2M, C3 and S100A8, A2M, C3 and HPX, A2M, C3 and SERPINF2, A2M, C3 and RBP4, A2M, LTF and MPO, A2M, LTF and HBA1, A2M, LTF and HP, A2M, LTF and S100A8, A2M, LTF and HPX, A2M, LTF and SERPINF2, A2M, LTF and RBP4, A2M, MPO and HBA1, A2M, MPO and HP, A2M, MPO and S100A8, A2M, MPO and HPX, A2M, MPO and SERPINF2, A2M, MPO and RBP4, A2M, HBA1 and HP, A2M, HBA1 and S100A8, A2M, HBA1 and HPX, A2M, HBA1 and SERPINF2, A2M, HBA1 and RBP4, A2M, HP and S100A8, A2M, HP and HPX, A2M, HP and SERPINF2, A2M, HP and RBP4, A2M, S100A8 and HPX, A2M, S100A8 and SERPINF2, A2M, S100A8 and RBP4, A2M, HPX and SERPINF2, A2M, HPX and RBP4, A2M, SERPINF2 and RBP4, C3, LTF and MPO, C3, LTF and HBA1, C3, LTF and HP, C3, LTF and S100A8, C3, LTF and HPX, C3, LTF and SERPINF2, C3, LTF and RBP4, C3, MPO and HBA1, C3, MPO and HP, C3, MPO and S100A8, C3, MPO and HPX, C3, MPO and SERPINF2, C3, MPO and RBP4, C3, HBA1 and HP, C3, HBA1 and S100A8, C3, HBA1 and HPX, C3, HBA1 and SERPINF2, C3, HBA1 and RBP4, C3, HP and S100A8, C3, HP and HPX, C3, HP and SERPINF2, C3, HP and RBP4, C3, S100A8 and HPX, C3, S100A8 and SERPINF2, C3, S100A8 and RBP4, C3, HPX and SERPINF2, C3, HPX and RBP4, C3, SERPINF2 and RBP4, LTF, MPO and HBA1, LTF, MPO and HP, LTF, MPO and S100A8, LTF, MPO and HPX, LTF, MPO and SERPINF2, LTF, MPO and RBP4, LTF, HBA1 and HP, LTF, HBA1 and S100A8, LTF, HBA1 and HPX, LTF, HBA1 and SERPINF2, LTF, HBA1 and RBP4, LTF, HP and S100A8, LTF, HP and HPX, LTF, HP and SERPINF2, LTF, HP and RBP4, LTF, S100A8 and HPX, LTF, S100A8 and SERPINF2, LTF, S100A8 and RBP4, LTF, HPX and SERPINF2, LTF, HPX and RBP4, LTF, SERPINF2 and RBP4, MPO, HBA1 and HP, MPO, HBA1 and S100A8, MPO, HBA1 and HPX, MPO, HBA1 and SERPINF2, MPO, HBA1 and RBP4, MPO, HP and S 100A8, MPO, HP and HPX, MPO, HP and SERPINF2, MPO, HP and RBP4, MPO, S100A8 and HPX, MPO, S100A8 and SERPINF2, MPO, S100A8 and RBP4, MPO, HPX and SERPINF2, MPO, HPX and RBP4, MPO, SERPINF2 and RBP4, HBA1, HP and S100A8, HBA1, HP and HPX, HBA1, HP and SERPINF2, HBA1, HP and RBP4, HBA1, S100A8 and HPX, HBA1, S100A8 and SERPINF2, HBA1, S100A8 and RBP4, HBA1, HPX and SERPINF2, HBA1, HPX and RBP4, HBA1, SERPINF2 and RBP4, HP, S100A8 and HPX, HP, S100A8 and SERPINF2, HP, S100A8 and RBP4, HP, HPX and SERPINF2, HP, HPX and RBP4, HP, SERPINF2 and RBP4, S100A8, HPX and SERPINF2, S100A8, HPX and RBP4, S100A8, SERPINF2 and RBP4, HPX, SERPINF2 and RBP4, A2M, C3, LTF and MPO, A2M, C3, LTF and HBA1, A2M, C3, LTF and HP, A2M, C3, LTF and S100A8, A2M, C3, LTF and HPX, A2M, C3, LTF and SERPINF2, A2M, C3, LTF and RBP4, A2M, C3, MPO and HBA1, A2M, C3, MPO and HP, A2M, C3, MPO and S100A8, A2M, C3, MPO and HPX, A2M, C3, MPO and SERPINF2, A2M, C3, MPO and RBP4, A2M, C3, HBA1 and HP, A2M, C3, HBA1 and S100A8, A2M, C3, HBA1 and HPX, A2M, C3, HBA1 and SERPINF2, A2M, C3, HBA1 and RBP4, A2M, C3, HP and S100A8, A2M, C3, HP and HPX, A2M, C3, HP and SERPINF2, A2M, C3, HP and RBP4, A2M, C3, S100A8 and HPX, A2M, C3, S100A8 and SERPINF2, A2M, C3, S100A8 and RBP4, A2M, C3, HPX and SERPINF2, A2M, C3, HPX and RBP4, A2M, C3, SERPINF2 and RBP4, A2M, LTF, MPO and HBA1, A2M, LTF, MPO and HP, A2M, LTF, MPO and S100A8, A2M, LTF, MPO and HPX, A2M, LTF, MPO and SERPINF2, A2M, LTF, MPO and RBP4, A2M, LTF, HBA1 and HP, A2M, LTF, HBA1 and S100A8, A2M, LTF, HBA1 and HPX, A2M, LTF, HBA1 and SERPINF2, A2M, LTF, HBA1 and RBP4, A2M, LTF, HP and S100A8, A2M, LTF, HP and HPX, A2M, LTF, HP and SERPINF2, A2M, LTF, HP and RBP4, A2M, LTF, S100A8 and HPX, A2M, LTF, S100A8 and SERPINF2, A2M, LTF, S100A8 and RBP4, A2M, LTF, HPX and SERPINF2, A2M, LTF, HPX and RBP4, A2M, LTF, SERPINF2 and RBP4, A2M, MPO, HBA1 and HP, A2M, MPO, HBA1 and S100A8, A2M, MPO, HBA1 and HPX, A2M, MPO, HBA1 and SERPINF2, A2M, MPO, HBA1 and RBP4, A2M, MPO, HP and S100A8, A2M, MPO, HP and HPX, A2M, MPO, HP and SERPINF2, A2M, MPO, HP and RBP4, A2M, MPO, S100A8 and HPX, A2M, MPO, S100A8 and SERPINF2, A2M, MPO, S100A8 and RBP4, A2M, MPO, HPX and SERPINF2, A2M, MPO, HPX and RBP4, A2M, MPO, SERPINF2 and RBP4, A2M, HBA1, HP and S100A8, A2M, HBA1, HP and HPX, A2M, HBA1, HP and SERPINF2, A2M, HBA1, HP and RBP4, A2M, HBA1, S100A8 and HPX, A2M, HBA1, S100A8 and SERPINF2, A2M, HBA1, S100A8 and RBP4, A2M, HBA1, HPX and SERPINF2, A2M, HBA1, HPX and RBP4, A2M, HBA1, SERPINF2 and RBP4, A2M, HP, S100A8 and HPX, A2M, HP, S100A8 and SERPINF2, A2M, HP, S100A8 and RBP4, A2M, HP, HPX and SERPINF2, A2M, HP, HPX and RBP4, A2M, HP, SERPINF2 and RBP4, A2M, S100A8, HPX and SERPINF2, A2M, S100A8, HPX and RBP4, A2M, S100A8, SERPINF2 and RBP4, A2M, HPX, SERPINF2 and RBP4, C3, LTF, MPO and HBA1, C3, LTF, MPO and HP, C3, LTF, MPO and S100A8, C3, LTF, MPO and HPX, C3, LTF, MPO and SERPINF2, C3, LTF, MPO and RBP4, C3, LTF, HBA1 and HP, C3, LTF, HBA1 and S100A8, C3, LTF, HBA1 and HPX, C3, LTF, HBA1 and SERPINF2, C3, LTF, HBA1 and RBP4, C3, LTF, HP and S100A8, C3, LTF, HP and HPX, C3, LTF, HP and SERPINF2, C3, LTF, HP and RBP4, C3, LTF, S100A8 and HPX, C3, LTF, S100A8 and SERPINF2, C3, LTF, S100A8 and RBP4, C3, LTF, HPX and SERPINF2, C3, LTF, HPX and RBP4, C3, LTF, SERPINF2 and RBP4, C3, MPO, HBA1 and HP, C3, MPO, HBA1 and S100A8, C3, MPO, HBA1 and HPX, C3, MPO, HBA1 and SERPINF2, C3, MPO, HBA1 and RBP4, C3, MPO, HP and S100A8, C3, MPO, HP and HPX, C3, MPO, HP and SERPINF2, C3, MPO, HP and RBP4, C3, MPO, S100A8 and HPX, C3, MPO, S100A8 and SERPINF2, C3, MPO, S100A8 and RBP4, C3, MPO, HPX and SERPINF2, C3, MPO, HPX and RBP4, C3, MPO, SERPINF2 and RBP4, C3, HBA1, HP and S100A8, C3, HBA1, HP and HPX, C3, HBA1, HP and SERPINF2, C3, HBA1, HP and RBP4, C3, HBA1, S100A8 and HPX, C3, HBA1, S100A8 and SERPINF2, C3, HBA1, S100A8 and RBP4, C3, HBA1, HPX and SERPINF2, C3, HBA1, HPX and RBP4, C3, HBA1, SERPINF2 and RBP4, C3, HP, S100A8 and HPX, C3, HP, S100A8 and SERPINF2, C3, HP, S100A8 and RBP4, C3, HP, HPX and SERPINF2, C3, HP, HPX and RBP4, C3, HP, SERPINF2 and RBP4, C3, S100A8, HPX and SERPINF2, C3, S100A8, HPX and RBP4, C3, S100A8, SERPINF2 and RBP4, C3, HPX, SERPINF2 and RBP4, LTF, MPO, HBA1 and HP, LTF, MPO, HBA1 and S100A8, LTF, MPO, HBA1 and HPX, LTF, MPO, HBA1 and SERPINF2, LTF, MPO, HBA1 and RBP4, LTF, MPO, HP and S100A8, LTF, MPO, HP and HPX, LTF, MPO, HP and SERPINF2, LTF, MPO, HP and RBP4, LTF, MPO, S100A8 and HPX, LTF, MPO, S100A8 and SERPINF2, LTF, MPO, S100A8 and RBP4, LTF, MPO, HPX and SERPINF2, LTF, MPO, HPX and RBP4, LTF, MPO, SERPINF2 and RBP4, LTF, HBA1, HP and S100A8, LTF, HBA1, HP and HPX, LTF, HBA1, HP and SERPINF2, LTF, HBA1, HP and RBP4, LTF, HBA1, S100A8 and HPX, LTF, HBA1, S 100A8 and SERPINF2, LTF, HBA1, S 100A8 and RBP4, LTF, HBA1, HPX and SERPINF2, LTF, HBA1, HPX and RBP4, LTF, HBA1, SERPINF2 and RBP4, LTF, HP, S100A8 and HPX, LTF, HP, S100A8 and SERPINF2, LTF, HP, S100A8 and RBP4, LTF, HP, HPX and SERPINF2, LTF, HP, HPX and RBP4, LTF, HP, SERPINF2 and RBP4, LTF, S100A8, HPX and SERPINF2, LTF, S100A8, HPX and RBP4, LTF, S100A8, SERPINF2 and RBP4, LTF, HPX, SERPINF2 and RBP4, MPO, HBA1, HP and S100A8, MPO, HBA1, HP and HPX, MPO, HBA1, HP and SERPINF2, MPO, HBA1, HP and RBP4, MPO, HBA1, S100A8 and HPX, MPO, HBA1, S100A8 and SERPINF2, MPO, HBA1, S100A8 and RBP4, MPO, HBA1, HPX and SERPINF2, MPO, HBA1, HPX and RBP4, MPO, HBA1, SERPINF2 and RBP4, MPO, HP, S100A8 and HPX, MPO, HP, S100A8 and SERPINF2, MPO, HP, S100A8 and RBP4, MPO, HP, HPX and SERPINF2, MPO, HP, HPX and RBP4, MPO, HP, SERPINF2 and RBP4, MPO, S100A8, HPX and SERPINF2, MPO, S100A8, HPX and RBP4, MPO, S100A8, SERPINF2 and RBP4, MPO, HPX, SERPINF2 and RBP4, HBA1, HP, S100A8 and HPX, HBA1, HP, S100A8 and SERPINF2, HBA1, HP, S100A8 and RBP4, HBA1, HP, HPX and SERPINF2, HBA1, HP, HPX and RBP4, HBA1, HP, SERPINF2 and RBP4, HBA1, S100A8, HPX and SERPINF2, HBA1, S100A8, HPX and RBP4, HBA1, S100A8, SERPINF2 and RBP4, HBA1, HPX, SERPINF2 and RBP4, HP, S100A8, HPX and SERPINF2, HP, S100A8, HPX and RBP4, HP, S100A8, SERPINF2 and RBP4, HP, HPX, SERPINF2 and RBP4, and S100A8, HPX, SERPINF2 and RBP4. All combinations indicated herein above have an area under the curve for controls versus CRC of at least 0.81.

Any of the combinations as indicated herein above may be combined with plasminogen (PLG), histidine-rich glycoprotein (HRG), complement component C9 (C9), plasma protease C1 inhibitor (SERPING1), serum amyloid P-component, Ig gamma-1 chain C region, Ig gamma-2 chain C region, alpha-1-acid glycoprotein 1, and/or alpha-1-acid glycoprotein 2. The inclusion of at least one of plasminogen (PLG), histidine-rich glycoprotein (HRG), complement component C9 (C9), plasma protease C1 inhibitor (SERPING1), serum amyloid P-component, Ig gamma-1 chain C region, Ig gamma-2 chain C region, alpha-1-acid glycoprotein 1, and/or alpha-1-acid glycoprotein 2 may increase sensitivity and/or specificity of a test for discriminating stool from a colorectal cancer patient from a control, especially for discriminating stool from an early stage colorectal cancer patient, such as for discriminating advanced adenocarcinomas from a control.

A further set of protein expression molecules comprises two or more expression products from SERPINF2, S100A8, S100A9, C3, TF, LTF, HBB, HPX, HBA1, HP, MPO, CDA, AZU1, RBP4, C5, FN1, and GSR, especially SERPINF2 and S100A8, preferably SERPINF2, S100A8 and HBA1 as such or in combination with C3. It was surprisingly found that, although the markers SERPINF2 and S100A8 or S100A8/A9 were not amongst the best individual markers for discriminating AA and/or CRC from controls, together they provided a high discriminating power over a large set of samples. In addition, a combination of SERPINF2 and S100A8 or S100A8/A9 was found to specifically improve to the results obtained with HBA1, resulting in a high sensitivity of a marker panel comprising SERPINF2, HBA1 and S100A8 or S100A8/A9, especially at high specificity (i.e. 95%).

A preferred set of genes for discriminating advanced adenomas from controls comprises S100A8 and RBP4, S100A8, RBP4 and HPX, S100A8, RBP4, HPX and HBD, S100A9 and C3, S100A9, C3 and SERPINF2, S100A9, C3, SERPINF2 and HPX.

A preferred set of genes for discriminating both colorectal cancers and advanced adenomas from controls comprises HP and SERPINF2, HP, SERPINF2 and HBD, HP, SERPINF2, HBD and PSMA5, C3 and HBB, C3, HBB and S100A8, C3, HBB, S100A8 and S100A9.

A preferred set of genes for discriminating colorectal cancers from controls comprises C3 and LTF, C3, LTF and HBA1, C3, LTF, HBA1 and HP, HP and A2M, HP, A2M and MPO, HP, A2M, MPO and CDA.

### 5.6 Kit for performing the method

The invention further provides a kit and the use of a kit as claimed, for determining whether an individual is suffering from a colorectal cancerous growth, the kit comprising a device for collecting a test sample from said individual, preferably a stool sample, and reagents for directly or indirectly determining a level of expression of at least two protein expression molecules, preferably proteins, in said sample.

The kit for performing the method according to the invention may be selected from any suitable assay and data processing apparatus and equipment.

Said reagents for determining a level of at least two protein expression molecules preferably are reagents for an immunochemical assay.

It is preferred that the reagents for determining a level of expression of the claimed protein exression molecules include a receptacle that is coated antibodies against said claimed protein expression molecules, or monolithic material or microbeads that are coated with antibodies against said claimed protein expression molecules, preferably proteins, allowing detection of a level of expression of said claimed protein expression molecules.

Disclosed but not claimed is that a receptacle preferably is an array, comprising a solid support and antibodies against said at least two protein expression molecules in an arrayed format that are immobilized on the solid support. The solid support is typically glass or a polymer, the most commonly used polymers being cellulose, polyacrylamide, nylon, polystyrene, polyvinyl chloride or polypropylene. The solid support may be in the form of tubes, beads, discs, silicon chips, microplates, polyvinylidene difluoride (PVDF) membrane, nitrocellulose membrane, nylon membrane, other porous membrane, non-porous membrane (e.g. plastic, polymer, polymethylmethacrylaat, silicon), a plurality of polymeric pins, or a plurality of microtitre wells, or any other surface suitable for immobilising proteins, antibodies and other suitable molecules and/or conducting an immunoassay. By using well-known techniques, such as contact or non-contact printing, masking or photolithography, the location of each spot on said solid support can be defined.

Said monolithic material or microbeads are preferably coated with antibodies against at least two protein expression molecules, more preferably at least three protein expression molecules, more preferably at least four protein expression molecules, most preferably at least five protein expression molecules, such as six protein expression molecules, seven protein expression molecules and ten protein expression molecules.

Said monolithic material or microbeads coated with multiple antibodies enable simultaneous detection of multiple protein expression molecules. The simultaneous analysis is cost effective and amenability to high-throughput/automation.

The invention further provides a use of a kit according to the invention for determining whether an individual is suffering from a colorectal cancerous growth.

### 5.7 Treatment

The identification of the biomarkers indicated herein above allows not only the detection of advanced adenomas and colorectal cancer, but also enables methods of treating colorectal cancers. Early diagnosis of colorectal cancer often allows for curative surgical removal of the tumour, whereas later diagnosis may result in a (chemo)therapeutic treatment. Said treatment preferably comprises colonoscopy if said individual is classified as having a colorectal cancerous growth, which may be followed by removal of at least part of the cancerous growth if presence of a cancerous growth is confirmed by colonoscopy.

Therefore, provided is a method of assigning or prescribing treatment to an individual suffering from colorectal cancer, said method comprising (a) classifying said individual as having a cancerous growth or as not having a cancerous growth according to a method of the invention, (b) assigning treatment comprising colonoscopy if said individual is classified as having said cancerous growth.

A preferred method further comprises removal of at least part of the cancerous growth if presence of a cancerous growth is confirmed by colonoscopy. Said treatment following colonoscopy preferably comprises surgery and/or (chemo)therapy, such as by assigning or administering 5-fluoruracil (5-FU), preferably in combination with leucovorin, or by assigning or administering capecitabine and/or oxaliplatin and/or irinotecan.

Detection of an advanced adenoma using a method of the invention, preferably is followed by colonoscopy and removal of at least part of the cancerous growth, preferably all of the cancerous growth.

Detection of a colorectal cancer using a method of the invention, preferably is followed by colonoscopy and removal of at least part of the cancerous growth, followed by surgery and/or (chemo)therapeutic treatment.

Therapeutic agents used to treat colorectal cancer include monoclonal antibodies, small molecule inhibitors and chemotherapeutic agents.

Typical therapeutic monoclonal antibodies include but are not limited to bevacizumab, cetuximab or panitumumab. Typical small molecule inhibitors include but are not limited to eriotinib, sorafenib or alisertib. Typical chemotherapeutic agents include but are not limited to 5-fluoruracil (5-FU), preferably further in combination with leucovorin, capecitabine, irinotecan and/or oxaliplatin. A preferred treatment comprises 5-FU, in combination with leucovorin and oxaliplatinin or 5-FU, in combination with leucovorin and irinotecan.

A further preferred treatment comprises capecitabine. Capecitabine may be used as adjuvant treatment, as monotherapy, or in combination with other agents for advanced or metastatic disease. Capecitabine may be used with either irinotecan or oxaliplatin, or used to replace 5-FU in any one of the above indicated combination treatments.

Any of the above indicated combinations comprising 5-FU or capecitabine may be combined with one or more of cetuximab, bevacizumab and panitumumab.

Combination therapies of, for example, a therapeutic monoclonal antibody and a small molecule inhibitor may be used. Thus, any combination of two or more of a monoclonal antibody, a small molecule inhibitor and a chemotherapeutic agent is envisaged.

### 5.8 General

For the purpose of clarity and a concise description, features are described herein as part of the same or separate aspects and preferred embodiments thereof, however, it will be appreciated that the scope of the invention may include embodiments having combinations of all or some of the features described.

The invention will now be illustrated by the following examples, which are provided by way of illustration and not of limitation. The scope of protection is defined by the appended claims.

### 6. EXAMPLES

### Example 1

### Methods

### Sample series

Written informed consent was obtained from all subjects who provided stool samples. The study was conducted in compliance with the institutional ethical regulations. See Table 1 for clinicopathological characteristics.

### Sample series 1:

Twenty-two stool subsamples (12 from CRC patients, 10 from subjects without colorectal neoplasia) were collected from a colonoscopy-controlled referral population between 2003 and 2006 at the VU University Medical Center in Amsterdam, The Netherlands. Samples were collected before colonoscopy or prior to surgical resection. At collection, stool samples were immediately stored at 4°C and transferred to -20°C within 36 hours. ~1 g stool was sampled from each stool specimen for protein extraction, which was done as described before 1 with few adaptations. In short, samples were homogenized in a two-fold excess volume of PBS by vortexing and centrifuged at 4°C; for 15 minutes at 16.000 G. The supernatants were centrifuged once more at 4°C for 10 minutes at full speed. Following the last spin cycle the supernatants were cleaned from remaining particles by filtering through a 0.22 µM PVDF filter (Merck Millipore, Billerica, MA, USA). Finally, the samples were concentrated to approximately 200 µl using a 3 kDa cut-off filter (Amicon Ultra, Merck Millipore, Billerica, MA, USA).

### Sample series 2:

Whole stool samples from 293 individuals diagnosed with CRC (n=81) advanced adenoma (AA; n=40), non-advanced adenomas (n=43), and individuals without colorectal neoplasia (n=129) were collected from a colonoscopy-controlled referral population at multiple centers in the Netherlands and Germany between 2005 and 2012. FIT data were not available for these samples.

Whole stool samples were collected prior to colonoscopy and before the start of laxative treatment preceding colonoscopy and processed according to standard procedures. All samples were collected according to a uniform standard operating procedure. Stool stabilization buffer (Exact Sciences, Madison, WI, USA) was

**Table 1: clinicopathological characteristics of stool sample series 1 and 2**

| | Sample series 1 n=22 | | | | Sample series 2 n=291* | | | | | | | |
|---|---|---|---|---|---|---|---|---|---|---|---|---|
| | Normal controls (n=10) | | CRC (n=12) | | Normal controls (n=129) | | Adenomas (n=43) | | Advanced adenomas (n=40) | | CRC (n=79) | |
| **Age mean in yrs (range)** | 66.7 (58-75) | | 76.5 (59-86) | | 57.8 (38-87) | | 65.1 (42-85) | | 65.8 (43-84) | | 68.6 (42-87) | |

| **Gender n(%)** | | | | | | | | | | | | |
|---|---|---|---|---|---|---|---|---|---|---|---|---|
| Male | 5 | 50% | 7 | 58% | 55 | 43% | 25 | 58% | 21 | 53% | 40 | 51% |
| Female | 5 | 50% | 5 | 42% | 74 | 57% | 18 | 42% | 19 | 48% | 39 | 49% |

| **Location** | | | | | | | | | | | | |
|---|---|---|---|---|---|---|---|---|---|---|---|---|
| right | - | - | 8 | 67% | - | - | 17 | 40% | 16 | 40% | 8 | 10% |
| left | - | - | 4 | 33% | - | - | 23 | 53% | 22 | 55% | 26 | 33% |
| left and right | - | - | 0 | 0% | - | - | 3 | 7% | 1 | 3% | 0 | 0% |
| unknown | - | - | 0 | 0% | - | - | 0 | 0% | 1 | 3% | 45 | 57% |
| **Lesion size mean in mm (range)** | - | | 47.0 (15-70) | | - | | 4.2 (1-8) | | 13.4 (2-40) | | 33.9 (6-96) | |

| **Adenomas: histological type n(%)** | | | | | | | | | | | | |
|---|---|---|---|---|---|---|---|---|---|---|---|---|
| tubular | - | - | - | - | - | - | 43 | 100 % | 12 | 30% | - | - |
| tubulovillous | - | - | - | - | - | - | 0 | 0% | 24 | 60% | - | - |
| villous | - | - | - | - | - | - | 0 | 0% | 3 | 8% | - | - |
| unknown | - | - | - | - | - | - | 0 | 0% | 1 | 3% | - | - |

| **Adenomas: dysplasia n(%)** | | | | | | | | | | | | |
|---|---|---|---|---|---|---|---|---|---|---|---|---|
| Low-grade | - | - | - | - | - | - | 43 | 100 % | 31 | 78% | - | - |
| High-grade | - | - | - | - | - | - | 0 | 0% | 8 | 20% | - | - |
| unknown | - | - | - | - | - | - | - | - | 1 | 3% | - | - |

| **Carcinomas: UICC stage n(%)** | | | | | | | | | | | | |
|---|---|---|---|---|---|---|---|---|---|---|---|---|
| I | - | - | 3 | 25% | - | - | - | - | - | - | 25 | 32% |
| II | - | - | 4 | 33% | - | - | - | - | - | - | 27 | 34% |
| III | - | - | 5 | 42% | - | - | - | - | - | - | 13 | 16% |
| IV | - | - | 0 | 0% | - | - | - | - | - | - | 13 | 16% |
| unknown | - | - | 0 | 0% | - | - | - | - | - | - | 1 | 1% |

| | | | | | | | | | | | | |
|---|---|---|---|---|---|---|---|---|---|---|---|---|
| * Data acquisition was not successful for two CRC samples in series 2, hence, the initial total number of 293 stool samples reduced to 291 for downstream analyses n = number of samples; CRC: Colorectal cancer - = not applicable | | | | | | | | | | | | |

added to the stool sample by the subject immediately after defecation, processed in the lab with a final stool:buffer w/v ratio of 1:7 within 72 hours, and stored at -80°C until use. Protein extracts were prepared as described for sample series 1 using 2 ml of homogenized stool sample as starting material.

### Sample series 3:

FIT samples were obtained prior to colonoscopy from 72 individuals diagnosed with CRC (n=14), AA (n=16), non-advanced adenomas (n=18) and individuals without colorectal neoplasia (n=24) at Kennemer Ghasthuis Hospital in Haarlem, The Netherlands, between 2012 and 2014. FIT fluid was taken from the sampling device with a needle and centrifuged at 4°C for 5 minutes at 13.200 RPM. The supernatant was used as input for further antibody-based analysis (see below; Meso Scale Discovery (MSD) Biomarker Assays.

### 1D-SDS gel electrophoresis and sample processing for nanoLC-MS/MS proteomics analysis

Equal amounts of protein (~30µg) from sample series 1 were loaded in an alternating order of control and CRC, and separated on precast 4-12% gradient SDS-PAGE gels (Invitrogen, Carlsbad, USA). The samples of sample series 2 were loaded in an alternating order of control, adenoma, advanced adenoma and CRC on a precast 12% SDS-PAGE gel and ran shortly into the gel until the proteins entered the running gel (Biorad, Veenendaal, the Netherlands). The gels were fixed in 50% ethanol containing 3% phosphoric acid, washed and stained overnight with Coomassie R-250. Gels were washed in ultrapure water (Merck Millipore, Billerica, MA, USA) and stored at 4°C until processing for in-gel tryptic digestion. The gels of sample series 1 were processed by cutting each lane into 10 equal individual bands while the samples of sample series 2 were cut out of the gel as a single band. Each of the bands was further processed into tryptic peptides as described before (Albrethsen et al., 2010. Molecular & cellular proteomics 9: 988-1005; Piersma et al., 2010. Journal of proteome research 9: 1913-22). Peptides were extracted and the volume of the (desalted) peptide fractions was reduced to 50 µl in a vacuum centrifuge. For technical reasons, approximately half of the peptide samples from sample series 2 were desalted as an extra cleanup step using Oasis HLB 96-well filter plates (Waters Chromatography B.V, Etten-Leur, The Netherlands), which did not affect protein quantification.

### nanoLC-MS/MS proteomics analysis

For both sample series 1 and 2, peptides were separated by an Ultimate 3000 nanoLC system (Dionex LC-Packings, Amsterdam, The Netherlands), equipped with a 20 cm x 75 µm ID fused silica column custom packed with 3 µm 120 Å ReproSil Pur C18 aqua (Dr Maisch GMBH, Ammerbuch-Entringen, Germany), as described previously (Piersma et al., 2010. Journal of proteome research 9: 1913-22). After injection, peptides were trapped at 6 pl/min on a 10 mm × 100 µm ID trap column packed with 5 µm 120 A ReproSil Pur C18 aqua at 2% buffer B (buffer A: 0.5% acetic acid in ultrapure water; buffer B: 80% ACN + 0.5% acetic acid in ultrapure water) and separated at 300 nl/min in a 10-40% buffer B gradient in 60 min (90 min inject-to-inject) in the 10-gel band for sample series 1 and 90 min (120 min inject-to-inject) in the single-shot for sample series 2. Samples were injected in alternating order of control-CRC for sample series 1 and control-adenoma-advanced adenoma-CRC for sample series 2.

For sample series 1, the eluting peptides were ionized at 1.7 kV in a Nanomate Triversa Chip-based nanospray source using a Triversa LC coupler (Advion, Ithaca, NJ) and detected on a LTQ-FT hybrid mass spectrometer (Thermo Fisher, Bremen, Germany). Intact masses were measured at resolution 50.000 in the ICR cell using a target value of 1 × 106 charges. In parallel, following an FT pre-scan, the top-5 peptide signals (charge-states 2+ and higher) were submitted to MS/MS in the linear ion trap (3 amu isolation width, 30 ms activation, 35% normalized activation energy, Q value of 0.25 and a threshold of 5000 counts). Dynamic exclusion was applied with a repeat count of 1 and an exclusion time of 30 seconds.

For sample series 2, the eluting peptides were ionized at a potential of +2 kVa into a Q Exactive mass spectrometer (Thermo Fisher, Bremen, Germany). Intact masses were measured at resolution 70.000 (at m/z 200) in the orbitrap using an AGC target value of 3 × 106 charges. The top 10 peptide signals (charge-states 2+ and higher) were submitted to MS/MS in the HCD (higher-energy collision) cell (4 amu isolation width, 25% normalized collision energy). MS/MS spectra were acquired at resolution 17.500 (at m/z 200) in the orbitrap using an AGC target value of 2 × 105 charges and an underfill ratio of 0.1%. Dynamic exclusion was applied with a repeat count of 1 and an exclusion time of 30 s. For two CRC samples from sample series 2 data acquisition was not successful.

### Database searching

MS/MS spectra were searched against the Uniprot human reference proteome fasta file, release January 2014, no fragments; 61552 entries using MaxQuant 1.4.1.2 (Cox and Mann, 2008. Nature Biotech 26: 1867-72). Enzyme specificity was set to trypsin and up to two missed cleavages were allowed. Cysteine carboxamidomethylation (Cys, +57.021464 Da) was treated as fixed modification and methionine oxidation (Met+15.994915 Da) and N-terminal acetylation (N-terminal, +42.010565 Da) as variable modifications. Peptide precursor ions were searched with a maximum mass deviation of 4.5 ppm and fragment ions with a maximum mass deviation of 20 ppm (default MaxQuant settings). Peptide and protein identifications were filtered at an FDR of 1% using the decoy database strategy. Proteins that could not be differentiated based on MS/MS spectra alone were grouped to protein groups that are referred to as the first protein in that group (default MaxQuant settings). Protein abundance was quantified by label free spectral counting (Liu et al., 2004. Anal Chem 76: 4193-201).

### Antibody-based assays

FIT fluids from sample series 3 were analyzed with antibody-based assays. MULTI-SPOT 96 4-Spot Prototype Human 4-plex plates pre-coated with capture antibodies directed against A2M, Adiponectin, RBP4 and MPO (N45ZA-1) and corresponding kit reagents were purchased from MSD (Rockville, USA). All solutions and protocols were prepared according to the manufacturer's instructions. The incubation time of the undiluted FIT fluid sample on the plate and the subsequent incubation of the detection antibody was for two 2 hours at room temperature with vigorous shaking. Standard curves were prepared in FIT buffer (Eiken Chemical Co.) using kit calibrators. After washing 3 more times and subsequent addition of 150 µl diluted read buffer, plates were immediately measured by electrochemiluminescence detection on the MSD SECTOR Imager 2400.

Data was analyzed using the MSD Discovery Workbench 4.0 software by application of a 4-parameter logistic curve-fitting algorithm including a 1/Y2 weighting function in order to generate standard curves for the calculation of the analyte concentration in the FIT fluid samples.

### Statistical Analysis

Statistical analyses were performed in the computing environment R version 3.1.1, including the packages rpart, pROC, gplots, and ggplot2 (Wickham H., 2009. ggplot2: Elegant Graphics for Data Analysis. : Springer-Verlag New York; Warnes et al., 2015. R package version 2.17.0; Robin et al., 2011. BMC Bioinformatics 12: 77; Therneau et al., 2010. R package version 41-10). Spectral counts were subjected to global normalization (Pham et al., 2010. Bioinformatics (Oxford, England) 26: 363-9), i.e. by dividing the counts per protein by the sum of all counts per sample and multiplying by the average sum across all samples. Hierarchical clustering was performed on log2 (normalized expression values +1) using the Euclidean distance for sample clustering, Spearman distance for protein clustering, and complete linkage in both clusterings. Heatmaps show the normalized to zero mean unit variance (z-scores) for individual proteins. Univariate differential abundance analysis was performed using the beta-binomial test, which takes into account the within-sample variation and the between-sample variation in a single statistical model (Pham et al., 2010. Bioinformatics (Oxford, England) 26: 363-9).

Proteins consistently more abundant in CRC samples compared to controls in sample series 1 and 2 (Benjamini Hochberg corrected P-value Q≤ 0.05) were the input for the selection of specific biomarker panels, using two complementary multivariate approaches on sample series 2, i.e. logistic regression (exhaustive search) and CART analysis.

### Logistic regression

All the protein combinations of up to four proteins were enumerated by exhaustive search, and the corresponding quadratic logistic regression models were produced to classify (Surinova et al., 2015. EMBO Mol Med 7: 1166-78) individuals into either CRCs versus controls, CRCs and AAs versus controls, or AAs versus controls. Controls consisted of 129 individuals without colorectal neoplasia. We also repeated the analyses with a control group including the 43 non-advanced adenoma samples.

Receiver operating characteristic (ROC) analysis was used to evaluate the performance of protein panels to discriminate lesions (AAs and/or CRCs) from controls by calculating the area under the ROC curve (AUC). To get a better estimate of the AUCs and P-values in ROC curve comparisons (protein panel versus hemoglobin), 1000 bootstrap replications were used. To test the statistical significance of the difference between the AUCs, the pROC bootstrap method was selected, which performs a non-parametric stratified resampling with the percentile method. The AUCs were used to rank protein combinations.

### CART analysis

Classification and regression tree analysis (CART) was performed to select a model of 3 to 6 proteins that performed best in classifying individuals into the groups CRCs versus controls, CRCs and AAs versus controls, or AA versus controls. Controls consisted of 129 individuals without colorectal neoplasia. We also repeated the analyses with a control group including the 43 non-advanced adenoma samples. CART is a non-parametric regression approach that naturally incorporates non-linear covariate effects as well as interaction between covariates. Because the resulting trees are with certainty too complex (thereby overfitting the data), the second part of the procedure consists of cross-validation to trim back the full tree. In the three classification problems in our study, it was chosen to trim back the tree to a maximum of 3 to 6 proteins (Breiman et al., 1983. Classification and Regression Trees. Wadsworth, Belmont Ca). The performance of the resulting protein trees was established using 10-fold cross-validation.

ROC analysis was used to evaluate the performance of the returned CART models (biomarker panels) to discriminate lesions (AAs and/or CRCs) from controls by calculating the AUC. To get a better estimate of the AUCs and P-values in ROC curve comparisons (protein panel versus hemoglobin), 1000 bootstrap replications were used. To test the statistical significance of the difference between the AUCs, the pROC bootstrap method was selected, which performs a non-parametric stratified resampling with the percentile method.

The returned CART model (biomarker panel) for each comparison, as well as the 10 different panels per comparison that resulted from the 10-fold cross-validation procedure were recorded. To test the statistical significance of the difference in sensitivity between any marker panel and hemoglobin, at 95% specificity, the McNemar test was used. Wilcoxon Rank Sum test was used to test for statistical significance of the protein levels between CRCs and controls as measured with an antibody-based method on FIT samples.

Linear regression was used to test whether age or gender were confounders in the relation between protein abundancy and the presence or absence of an advanced lesion. Spearman rank correlation, Mann-Whitney or Kruskal-Wallis tests were used to test the relation between protein abundancy and tumor characteristics such as tumor size, tumor location, tumor stage (CRC), histology (AA) or grade of dysplasia (AA).

### Results

### Proteomics analysis of human stool samples

A total of 468 human proteins were identified in sample series 1 (data not shown). Spectral counts for the α- and β-chain of hemoglobin, known to be present in equal amounts (Schechter, 2008. Blood 112: 3927-38), showed a strong correlation (rho=0.95, P=2.2e-11; data not shown). Likewise, the S100A8 and S100A9 calprotectin subunits strongly correlated (rho=0.91, P=9.3e-09). Unsupervised cluster analysis revealed that most CRC stool samples had a different protein profile than those of control subjects (data not shown). Since these results confirm the feasibility of quantifying CRC-specific human proteins in stool samples, the analysis was extended to the second, larger series of samples.

Subsequent analysis of 291 stool samples (Table 1) revealed the presence of a total of 733 human proteins (data not shown). Also in this sample series, protein levels of hemoglobin α and β as well as the calprotectin subunits were highly correlated (rho=0.94, P=2.2e-16 and rho=0.8, P=2.2e-16, respectively). Again, the CRC stool samples had a different protein profile than control stool samples (Figure 1A and 1B).

In sample series 1 and 2 combined, a total of 884 human proteins were detected, of which 373 (45%) were common to both series (Figure 1C).

### Proteins discriminating CRCs from controls

Differential abundance analysis (CRCs versus controls; Fold change>0, P-value≤0.05) yielded 93 and 213 proteins in sample series 1 and 2, respectively, of which 55 were common. This list of 55 proteins reduced to 29 after correction for multiple testing (i.e. Q-value≤0.05) (Table 2). These 29 proteins included hemoglobin sub-units HBA1, HBB and HBD. C3, A2M and HP individually discriminated CRCs from controls significantly better than hemoglobin (HBA1), based on a higher AUC from ROC analysis. Since population-based screening requires high sensitivity combined with high specificity, specificity was fixed at 95% to evaluate the corresponding sensitivities. At a specificity of 95%, six proteins had a significantly higher sensitivity than HBA1, i.e. C3, A2M, HP, C5, FN1 and CP.

**Table 2: 29 candidate proteins; mean spectral counts per positive sample, fold changes and P/Q-values.**

| | | | **Sample series 1** | | | | | **Sample series 2** | | | | |
|---|---|---|---|---|---|---|---|---|---|---|---|---|
| | | | **Total number of positive samples (mean spectral counts per positive sample)** | | **Fold change^{a}** | **P-value^{a}** | **Q-value^{a}** | **Total number of positive samples (mean spectral count per positive sample)** | | **Fold change^{a}** | **P-value^{a}** | **Q-value^{a}** |
| **Gene Symbol** | **Entrez Gene Name** | **Uniprot Identifier** | **Controls N=10** | **CRCs N=12** | **CRCs *versus* Controls** | **CRCs *versus* Controls** | **CRCs *versus* Controls** | **Controls N=129** | **CRCs N=79** | **CRCs *versus* Controls** | **CRCs *versus* Controls** | **CRCs *versus* Controls** |
| A2M | alpha-2-macroglobulin | P01023 | 6 (10,7) | 12 (120.4) | 34.02 | 4.55E-06 | 1.5E-03 | 118 (21.1) | 79 (145.5) | 5.66 | 1.9E-24 | 3.1E-22 |
| S100A8 | S100 calcium binding protein A8 | P05109 | 8 (3,9) | 12 (10.8) | 3.90 | 8.26E-06 | 1.5E-03 | 128 (6.5) | 79 (15.6) | 1.80 | 1.6E-12 | 4.0E-11 |
| S100A9 | S100 calcium binding protein A9 | P06702 | 9 (2,8) | 12 (10.5) | 5.04 | 1.25E-05 | 1.5E-03 | 129 (9) | 79 (23.1) | 1.92 | 1.3E-10 | 2.5E-09 |
| CP | ceruloplasmin (ferroxidase) | P00450 | 1 (1) | 11 (32.9) | 583.48 | 1.21E-05 | 1.5E-03 | 38 (4.4) | 64 (21.8) | 11.08 | 4.6E-21 | 3.7E-19 |
| TF | transferrin | P02787 | 1 (1) | 11 (68.4) | 1227.20 | 2.33E-05 | 1.6E-03 | 21 (9.6) | 53 (61.2) | 21.85 | 1.9E-16 | 8.3E-15 |
| CAT | catalase | P04040 | 0 (0) | 9 (22.2) | ∞ | 2.00E-05 | 1.6E-03 | 48 (5.1) | 59 (20.3) | 5.31 | 2.7E-10 | 4.9E-09 |
| C9 | complement component 9 | P02748 | 0 (0) | 9 (3.4) | ∞ | 2.40E-05 | 1.6E-03 | 2 (1.5) | 12 (2.4) | 11.22 | 5.3E-04 | 2.5E-03 |
| LTF | lactotransferrin | P02788 | 2 (3,5) | 10 (34.3) | 80.52 | 3.38E-05 | 1.8E-03 | 109 (13.9) | 79 (57) | 3.75 | 1.1E-22 | 1.1E-20 |
| HBB | hemoglobin, beta | P68871 | 6 (10,5) | 12 (73.2) | 9.87 | 5.75E-05 | 2.4E-03 | 68 (12.1) | 77 (49.9) | 5.79 | 9.1E-25 | 2.2E-22 |
| HPX | hemopexin | P02790 | 0 (0) | 8 (14.8) | ∞ | 6.18E-05 | 2.4E-03 | 31 (5) | 59 (29.7) | 13.73 | 7.2E-17 | 3.3E-15 |
| HBA1 | hemoglobin, alpha 1 | P69905 | 2 (1) | 10 (46.7) | 79.91 | 7.88E-05 | 2.7E-03 | 52 (8.7) | 72 (36.2) | 7.93 | 5.0E-22 | 4.6E-20 |
| HP | haptoglobin | P00738 | 5 (3,8) | 11 (55.5) | 17.02 | 9.40E-05 | 2.9E-03 | 24 (9.9) | 69 (39.6) | 17.35 | 7.6E-32 | 5.5E-29 |
| GPI | glucose-6-phosphate isomerase | P06744 | 0 (0) | 8 (4.5) | ∞ | 1.64E-04 | 4.5E-03 | 60 (5.3) | 60 (12.9) | 2.82 | 9.7E-07 | 9.1E-06 |
| MPO | myeloperoxidase | P05164-2 | 0 (0) | 7 (6) | ∞ | 2.15E-04 | 5.0E-03 | 85 (8.7) | 77 (29.5) | 3.81 | 1.0E-18 | 5.4E-17 |
| HBD | hemoglobin, delta | P02042 | 0 (0) | 7 (13.4) | ∞ | 2.12E-04 | 5.0E-03 | 8(6) | 33 (7.7) | 8.16 | 3.5E-10 | 5.9E-09 |
| C3 | complement component 3 | P01024 | 4 (3,5) | 11 (99.8) | 68.01 | 2.90E-04 | 6.1E-03 | 103 (17.6) | 79 (112.5) | 6.59 | 4.2E-26 | 1.5E-23 |
| SERPINF2 | serpin peptidase inhibitor, clade F | P08697-2 | 1 (5) | 9 (4.3) | 13.21 | 4.15E-04 | 7.8E-03 | 93 (3) | 71 (10.6) | 3.21 | 4.0E-12 | 9.3E-11 |
| CDA | cytidine deaminase | P32320 | 0 (0) | 8 (1.4) | ∞ | 4.33E-04 | 7.8E-03 | 65 (1.5) | 63 (2.5) | 1.99 | 6.2E-05 | 3.9E-04 |
| FGG | fibrinogen gamma chain | P02679-2 | 0 (0) | 6 (10.8) | ∞ | 1.03E-03 | 1.5E-02 | 1 (1) | 20 (4.7) | 218.27 | 1.2E-08 | 1.5E-07 |
| AZU1 | azurocidin 1 | P20160 | 0 (0) | 6 (1.3) | ∞ | 1.12E-03 | 1.6E-02 | 63 (2.7) | 72 (6) | 3.07 | 3.7E-13 | 1.0E-11 |
| VTN | vitronectin | P04004 | 0 (0) | 6 (2.5) | ∞ | 1.78E-03 | 2.1E-02 | 3 (2.7) | 31 (5.1) | 38.14 | 1.3E-12 | 3.3E-11 |
| RBP4 | retinol binding protein 4, plasma | Q5VY30 | 2 (4) | 8 (5.6) | 7.37 | 1.98E-03 | 2.2E-02 | 37 (3) | 68 (8.1) | 5.62 | 3.2E-20 | 2.1E-18 |
| KNG1 | kininogen 1 | P01042-2 | 0 (0) | 5 (2) | ∞ | 2.08E-03 | 2.2E-02 | 2 (1) | 21 (2.4) | 39.18 | 8.0E-08 | 8.7E-07 |
| PSMA5 | proteasome (prosome, macropain) subunit, alpha type, 5 | P28066 | 0 (0) | 5 (1.8) | ∞ | 3.69E-03 | 3.4E-02 | 36 (2.3) | 52 (2.8) | 1.84 | 1.4E-05 | 1.0E-04 |
| C5 | complement component 5 | P01031 | 0 (0) | 5 (8.6) | ∞ | 4.11E-03 | 3.4E-02 | 11 (6.5) | 57 (10.4) | 13.04 | 2.1E-24 | 3.1E-22 |
| FN1 | fibronectin 1 | P02751-11 | 1 (2) | 8 (8.9) | 69.90 | 4.70E-03 | 3.7E-02 | 18 (5.1) | 61 (18.9) | 17.67 | 3.4E-24 | 4.2E-22 |
| LDHA | lactate dehydrogenase A | P00338 | 0 (0) | 5 (2.6) | ∞ | 5.66E-03 | 4.3E-02 | 24 (1.6) | 29 (3.2) | 2.29 | 1.4E-02 | 4.4E-02 |
| PRTN3 | proteinase 3 | P24158 | 1 (1) | 6 (2.8) | 22.25 | 6.17E-03 | 4.6E-02 | 72 (3.3) | 71 (6.6) | 2.42 | 3.0E-10 | 5.3E-09 |
| GSR | glutathione reductase | P00390-2 | 1 (1) | 7 (5.6) | 31.50 | 6.79E-03 | 4.9E-02 | 93 (3.6) | 70 (6.8) | 1.50 | 1.8E-03 | 7.4E-03 |

| | | | | | | | | | | | | |
|---|---|---|---|---|---|---|---|---|---|---|---|---|
| * Data acquisition was not successful for two CRC samples in series 2, hence, the initial total number of 293 stool samples reduced to 291 for downstream analyses n = number of samples; CRC: Colorectal cancer; LR = Logistic regression; CART: Classification and regression tree analysis - = not applicable ∞ = indefinitive | | | | | | | | | | | | |

### Biomarker panel selection

A panel of several biomarkers was sought that would perform better than hemoglobin. Logistic regression and CART analysis were applied to sample series 2; this series had adequate sample size and included stool samples from patients with (advanced) adenomas. Overall, combinations of four proteins yielded more discriminatory power than two- or three-protein combinations (data not shown).

### Logistic regression

The top-10 best performing four-protein panels, ranked according to Area Under the Curve (AUC), are shown in Table 3. All ten panels showed a significantly higher AUC than HBA1 alone (0.93-0.94 for the marker panels versus 0.88 for HBA1 alone), as well as a significantly higher sensitivity at 95% specificity (65-80% for the marker panels versus 43% for HBA1). The best performing panel as based on AUC consisted of C3, LTF, HBA1 and HP (Figure 2A). The top-10 best performing four-protein models together included 19 different proteins. The frequencies at which these individual proteins occurred in the top-10 best performing panels are shown in Figure 2B.

We performed the same analysis for advanced neoplasia (AAs plus CRCs) and AAs versus controls. The best performing panels consisted of HP, SERPINF2, HBD and PSMA5 for advanced neoplasia (Figure 2C), and S100A8, HPX, RBP4 and HBD for AAs (Figure 2E). The frequencies at which these individual proteins occurred in the top-10 best performing panels are shown in Figures 2D and 2F.

**Table 3. The top-10 best performing four-protein panels**

| **CRCs *vs* controls** | **Logistic regression models** | | | | | | |
|---|---|---|---|---|---|---|---|
| **protein 1** | **protein 2** | **protein 3** | **protein 4** | **AUC** | **p-value when compared to AUC of HBA1 alone (AUC of 0.88)** | **sensitivity at 95% specificity** | **p-value when compared to sensitivity at 95% specificity of HBA1 alone (43%)** |
| C3 | LTF | HBA1 | HP | 0.94 | 8.52E-03 | 71% | 7.20E-05 |
| C3 | HBA1 | HP | SERPIN F2 | 0.93 | 6.83E-03 | 68% | 3.30E-04 |
| C3 | HBB | HBA1 | SERPIN F2 | 0.93 | 6.26E-03 | 73% | 1.38E-05 |
| C3 | HP | SERPINF 2 | GSR | 0.93 | 1.44E-02 | 80% | 2.00E-07 |
| C3 | HBA1 | HP | AZU1 | 0.93 | 2.49E-02 | 68% | 7.94E-04 |
| LTF | HBA1 | SERPINF 2 | FGG | 0.93 | 1.73E-03 | 66% | 1.44E-04 |
| HBB | TF | MPO | C5 | 0.93 | 9.44E-03 | 68% | 5.23E-04 |
| C3 | CDA | PSMA5 | KNG1 | 0.93 | 3.10E-02 | 70% | 1.19E-04 |
| LTF | HBB | FN1 | RBP4 | 0.93 | 8.71E-03 | 65% | 5.35E-03 |
| C3 | HBB | HPX | C9 | 0.93 | 3.10E-02 | 78% | 1.82E-06 |

### Classification and regression tree (CART) analysis

CART analysis returns one selected model (biomarker panel) as output. The panel selected to discriminate between CRCs and controls consisted of HP, A2M, MPO and CDA, reaching an AUC of 0.94 and a sensitivity of 72% at 95% specificity (Figure 3A). The selected panels discriminating between advanced neoplasia and controls, and between AAs and controls consisted of C3, HBB, S100A8 and S100A9 (AUC=0.86, sensitivity of 67% at 95% specificity) and C3, S100A9, SERPINF2 and HPX, respectively (AUC=0.79, sensitivity of 45% at 95% specificity) (Figure 3C and 3E). Predicted accuracy of a CART model fitted on the full dataset would be overly optimistic, as the data would then be used both for fitting and testing. Therefore, we performed a tenfold cross-validation procedure to obtain an adjusted AUC to obtain the resulting top 10 panels for CRCs, advanced neoplasia and AAs versus controls. Frequencies of the individual proteins in the 10 panels for each comparison are shown in Figure 3B, 3D and 3F.

Screening populations include individuals with non-advanced adenomas, which are not the target lesions of a screening program. We therefore repeated the logistic regression and CART analyses with a control group including the 43 non-advanced adenoma samples. In short, the sensitivity at 95% specificity of the best performing biomarker panels in these analyses was 3-8% lower in the logistic regression and 3-4% lower or higher in the *CART* analyses than in the analyses without non-advanced adenomas in the control group.

### Top-performing proteins

In total, 17 proteins were included in the top-10 best performing biomarker panels in both the logistic regression and the CART analysis (Table 2). Of these, eleven proteins discriminated CRCs from controls (C3, MPO, AZU1, FN1, LTF, C5, RBP4, CDA, HP, HBB and HBA1), eight proteins discriminated advanced neoplasia from controls (C3, S100A8, S100A9, HBB, HP, SERPINF2, TF and GSR) and five proteins discriminated AAs from controls (SERPINF2, LTF, HPX, MPO and AZU1). Which of these combinations will perform best in screening practice will, amongst others, be determined by available assay technology, as well as availability and performance of antibodies against these proteins.

Age nor gender were confounding factors in the relation between protein abundance (spectral counts) and the presence of an advanced lesion (AA or CRC) for these proteins. The abundancy level of several of these proteins were positively correlated with tumor characteristics, such as larger tumor size, left-sided tumor location, more advanced CRC stage, and high-grade AA dysplasia.

### Biomarker detection in FIT sample

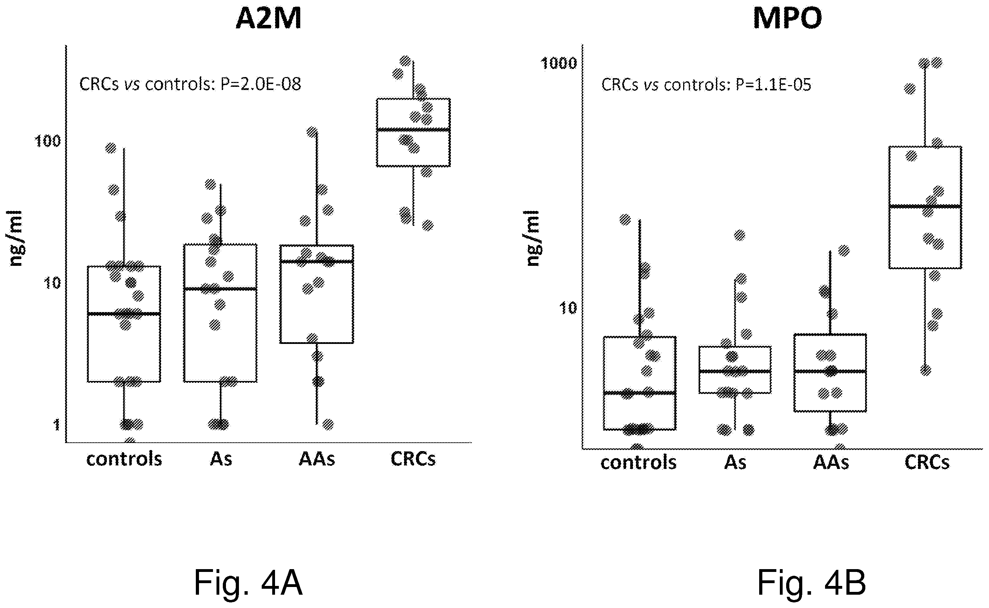

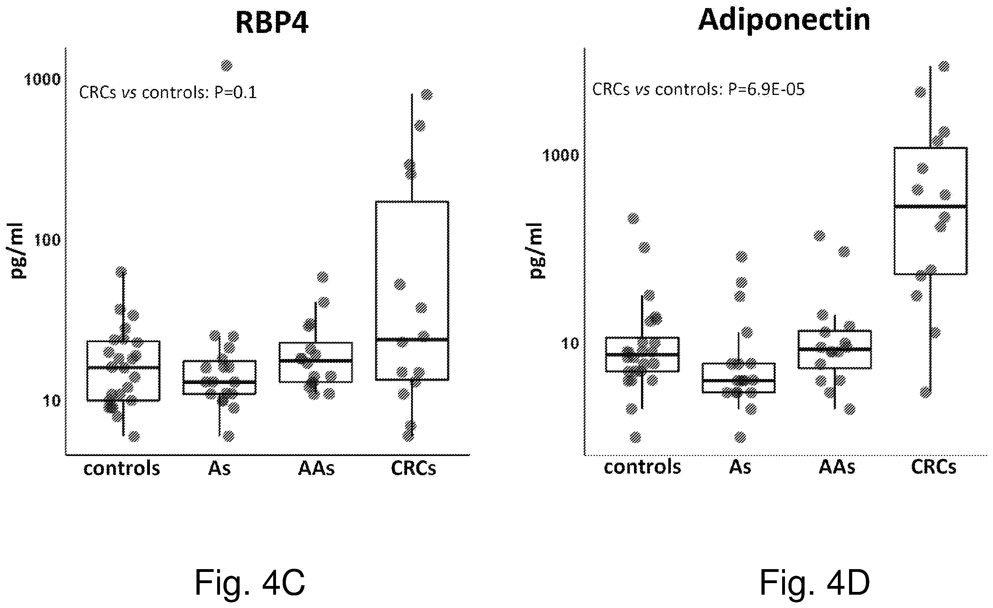

To obtain proof-of-concept that the candidate biomarkers identified by mass spectrometry in larger stool samples, can also be quantified in small stool sample volumes we performed an antibody-based evaluation on an independent series of FIT samples. An off-the-shelve antibody-based assay was available for four proteins we had identified, i.e. A2M, MPO, RBP4 and adiponectin. A2M, MPO and adiponectin were present at significantly higher concentrations in the FIT samples from CRC patients compared to controls (P<0.001; Figure 4).

### Example 2

### Samples and biomarker analysis

FIT samples were obtained from 1310 individuals prior to colonoscopy. FIT fluid was taken from the sampling device with a needle and used as input for further antibody-based analysis (see below; Meso Scale Discovery (MSD) *Biomarker* Assays).
Following biomarker analysis 1284 samples remained with good quality data, these included samples from individuals diagnosed with colorectal cancer (CRC) (n=37), advanced adenomas (AA) (n=165), non-advanced adenomas (n=251) and individuals without colorectal neoplasia (n=831). FIT fluids were analyzed with MSD antibody-based plate assays using antibodies directed against C3, HPT, RBP4, SERPINF2, A2M, HPX, MPO, Calprotectin (S100A8/A9), Hb and LTF. Concentrations were obtained through comparison to a calibrator (standard protein concentration). All measurements were performed in duplicate.

### CART analysis

Statistical analyses were performed in the computing environment R (R Development Core Team (2008). R: A language and environment for statistical computing. R Foundation for Statistical Computing, Vienna, Austria. ISBN 3-900051-07-0) and the analysis software SPSS (IBM). First, an average protein concentration was calculated over two duplicate measurements that were done used the MSD assays. This average concentration was the input for further analysis. Using the rpart package in R statistics, classification and regression tree analysis (CART) was performed to select a model of proteins that performed best in classifying individuals into the groups CRCs and AAs *versus* controls. CART is a non-parametric regression approach that naturally incorporates non-linear covariate effects as well as interaction between covariates.

Because the resulting full *CART* trees are with certainty too complex (thereby overfitting the data), the second part of the procedure consisted of cross-validation to trim back the full tree. Whereas pruning of a CART tree is usually guided by specifying the value of the complexity parameter, we pruned the tree by specifying the minimal number of samples per split (individuals), which was set at 50. The performance of the resulting protein trees was established using ROC analysis.

### Results

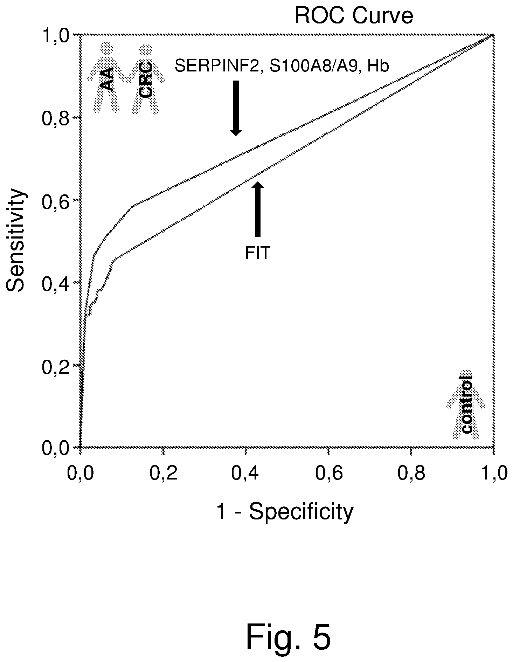

CART analysis resulted into a combination of three candidate biomarkers for the detection of Advanced Adenoma (AA) and a combination of AA and CRC, which outperformed FIT alone. These included Hb, SERPINF2 and S100A8/A9. The AUC of FIT was 0.70 and the AUC of the combination of three markers was 75.2. More importantly the sensitivity of the marker panel at a high specificity (i.e. 95%) was higher than that of the FIT, 51% versus 38%, respectively. See Figure 5.

From these data, it is concluded that a set of markers comprising SERPINF2 and S100A8, especially when supplemented with Hb or with Hb and S100A9, outperform FIT for detection of Advanced Adenoma (AA) and a combination of AA and CRC.

## Claims

1. A method for typing a sample of an individual suffering from a colorectal cancerous growth, or suspected of suffering therefrom, the method comprising
a. providing a stool sample comprising protein expression molecules from cancerous cells or suspected to comprise protein expression molecules from cancerous cells;
b. determining expression levels for said expression molecules of a set of genes in said sample; and
c. typing said sample on the basis of the expression levels determined for said expression molecules of the set of genes;
wherein said set of genes consists of SERPINF2, S100A8 and HBA1.

2. The method of claim 1, wherein said colorectal cancerous growth is an advanced adenoma.

3. The method according to claim 1 or claim 2, wherein said expression level is determined with an antibody or a functional part thereof directed against said protein expression molecule.

4. The method according to any of the previous claims, whereby said typing differentiates advanced adenoma cells from other cell types.

5. A kit consisting of reagents for directly or indirectly determining a level of expression of a set of proteins in a stool sample, consisting of reagents for an immunochemical assay, whereby said set of proteins consist of SERPINF2, S100A8 and HBA1.

6. Use of the kit according to claim 5 for determining expression levels of a set of genes consisting of SERPINF2, S100A8 and HBA1 in a stool sample.

7. A method for classifying an individual as having a colorectal cancerous growth, comprising
(a) providing a stool sample from the individual;
(b) extracting protein expression molecules from said stool sample;
(c) reacting said extracted protein expression molecules with different antibodies, directed against a set of extracted protein expression molecules consisting of expression molecules of SERPINF2, S100A8 and HBA1;
(d) quantifying reaction products between said antibodies and said extracted protein expression molecules;
(e) determining a level of expression of said extracted protein expression molecules, based on the quantified reaction products;
(f) comparing said determined expression levels with the expression levels of said extracted protein expression molecules in a reference;
(g) determining a similarity value between a level of expression of said extracted protein expression molecules in said individual and a level of expression of the extracted protein expression molecules in a patient not having a cancerous growth; and
(h) classifying said individual as having a cancerous growth if said similarity value is below a first similarity threshold value, and classifying said individual as not having a cancerous growth if said similarity value exceeds said first similarity threshold value.

8. 5-Fluoruracil (5-FU) for use in a method of treating an individual who is classified as having a cancerous growth according to claim 7 and presence of a cancerous growth was confirmed by colonoscopy.

9. 5-Fluoruracil (5-FU) for use according to claim 8, wherein said 5-FU is combined with leucovorin,

10. 5-Fluoruracil (5-FU) for use according to claim 8 or claim 9, wherein the method of treating comprises 5-FU, leucovorin and oxaliplatin, or 5-FU, leucovorin and irinotecan.

11. Capecitabine for use in a method of treating an individual who is classified as having a cancerous growth according to claim 7 and presence of a cancerous growth was confirmed by colonoscopy.

12. Capecitabine for use according to claim 11, wherein said capecitabine is combined with irinotecan and/or oxaliplatin.

## Patentansprüche

1. Verfahren zum Typisieren einer Probe eines Individuums, das an einem kolorektalen Krebswachstum leidet oder im Verdacht steht, daran zu leiden, wobei das Verfahren umfasst:
a. Bereitstellen einer Stuhlprobe, umfassend Proteinexpressionsmoleküle von Krebszellen oder im Verdacht stehend, Proteinexpressionsmoleküle von Krebszellen zu umfassen;
b. Bestimmen von Expressionsniveaus der Expressionsmoleküle eines Satzes von Genen in der Probe; und
c. Typisieren der Probe auf Basis der für die Expressionsmoleküle des Satzes von Genen bestimmten Expressionsniveaus;
wobei der Satz von Genen aus SERPINF2, S100A8 und HBA1 besteht.

2. Verfahren nach Anspruch 1, wobei das kolorektale Krebswachstum ein fortgeschrittenes Adenom ist.

3. Verfahren nach Anspruch 1 oder Anspruch 2, wobei das Expressionsniveau mit einem gegen das Proteinexpressionsmolekül gerichteten Antikörper oder einem funktionellen Teil davon bestimmt wird.

4. Verfahren nach einem der vorstehenden Ansprüche, wobei die Typisierung fortgeschrittene Adenomzellen von anderen Zelltypen unterschiedet.

5. Kit, bestehend aus Reagenzien zur direkten oder indirekten Bestimmung des Expressionsniveaus eines Satzes von Proteinen in einer Stuhlprobe, bestehend aus Reagenzien für einen immunchemischen Assay, wobei der Satz von Proteinen aus SERPINF2, S 100A8 und HBA1 besteht.

6. Verwendung des Kits nach Anspruch 5 zur Bestimmung der Expressionsniveaus eines Satzes von Genen, bestehend aus SERPINF2, S 100A8 und HBA1, in einer Stuhlprobe.

7. Verfahren zum Klassifizieren eines Individuums mit einem kolorektalen Krebswachstum, umfassend
(a) Bereitstellen einer Stuhlprobe des Individuums;
(b) Extrahieren von Proteinexpressionsmolekülen aus der Stuhlprobe;
(c) Umsetzen der extrahierten Proteinexpressionsmoleküle mit verschiedenen Antikörpern, die gegen einen Satz extrahierter Proteinexpressionsmoleküle gerichtet sind, der aus Expressionsmolekülen von SERPINF2, S100A8 und RBA1 besteht;
(d) Quantifizieren der Reaktionsprodukte zwischen den Antikörpern und den extrahierten Proteinexpressionsmolekülen;
(e) Bestimmen eines Expressionsniveaus der extrahierten Proteinexpressionsmoleküle auf Basis der quantifizierten Reaktionsprodukte;
(f) Vergleichen der bestimmten Expressionsniveaus mit den Expressionsniveaus der extrahierten Proteinexpressionsmoleküle in einer Referenz;
(g) Bestimmen eines Ähnlichkeitswertes zwischen einem Expressionsniveau der extrahierten Proteinexpressionsmoleküle in dem Individuum und einem Expressionsniveau der extrahierten Proteinexpressionsmoleküle in einem Patienten ohne Krebswachstum; und
(h) Klassifizieren des Individuums als mit Krebswachstum, wenn der Ähnlichkeitswert unter einem ersten Ähnlichkeitsschwellenwert ist, und Klassifizieren des Individuums als ohne Krebswachstum, wenn der Ähnlichkeitswert den ersten Ähnlichkeitsschwellenwert überschreitet.

8. 5-Fluoruracil (5-FU) zur Verwendung in einem Verfahren zur Behandlung eines Individuums, das nach Anspruch 7 als mit Krebswachstum klassifiziert ist und Vorhandensein eines Krebswachstums durch Koloskopie bestätigt wurde.

9. 5-Fluoruracil (5-FU) zur Verwendung nach Anspruch 8, wobei das 5-FU mit Leucovorin kombiniert ist.

10. 5-Fluoruracil (5-FU) zur Verwendung nach Anspruch 8 oder Anspruch 9, wobei das Behandlungsverfahren 5-FU, Leucovorin und Oxaliplatin oder 5-FU, Leucovorin und Irinotecan umfasst.

11. Capecitabin zur Verwendung in einem Verfahren zur Behandlung eines Individuums, das nach Anspruch 7 als mit Krebswachstum klassifiziert ist und Vorhandensein eines Krebswachstums durch Kolonoskopie bestätigt wurde.

12. Capecitabin zur Verwendung nach Anspruch 11, wobei das Capecitabin mit Irinotecan und/oder Oxaliplatin kombiniert ist.

## Revendications

1. Procédé de typage d'un échantillon d'un individu souffrant d'une croissance cancéreuse colorectale, ou suspecté de souffrir de celle-ci, le procédé comprenant les étapes consistant à
a. mettre à disposition un échantillon de selles comprenant des molécules d'expression protéiniques issues de cellules cancéreuses ou suspecté de comprendre des molécules d'expression protéiniques issues de cellules cancéreuses ;
b. déterminer les taux d'expression pour lesdites molécules d'expression d'un ensemble de gènes dans ledit échantillon ; et
c. typer ledit échantillon en se basant sur les taux d'expression déterminés pour lesdites molécules d'expression de l'ensemble de gènes ; dans lequel ledit ensemble de gènes consiste en SERPINF2, S100A8 et HBA1.

2. Procédé selon la revendication 1, dans lequel ladite croissance cancéreuse colorectale est un adénome avancé.

3. Procédé selon la revendication 1 ou la revendication 2, dans lequel ledit taux d'expression est déterminé avec un anticorps ou une partie fonctionnelle de celui-ci dirigé contre ladite molécule d'expression protéinique.

4. Procédé selon l'une quelconque des revendications précédentes, moyennant quoi ledit typage permet de différencier des cellules d'adénome avancé des autres types de cellules.

5. Kit consistant en des réactifs pour directement ou indirectement déterminer un taux d'expression d'un ensemble de protéines dans un échantillon de selles, consistant en des réactifs pour un dosage immunochimique, moyennant quoi ledit ensemble de protéines consiste en SERPINF2, S100A8 et HBA1.

6. Utilisation du kit selon la revendication 5 pour déterminer les taux d'expression d'un ensemble de gènes consistant en SERPINF2, S100A8 et HBA1 dans un échantillon de selles.

7. Procédé de classification d'un individu comme présentant une croissance cancéreuse colorectale, comprenant les étapes consistant à
(a) mettre à disposition un échantillon de selles de l'individu ;
(b) extraire des molécules d'expression protéiniques à partir dudit échantillon de selles ;
(c) faire réagir lesdites molécules d'expression protéiniques avec différents anticorps, dirigés contre un ensemble de molécules d'expression protéiniques extraites consistant en des molécules d'expression de SERPINF2, S100A8 et HBA1 ;
(d) quantifier des produits de réaction entre lesdits anticorps et lesdites molécules d'expression protéiniques extraites ;
(e) déterminer un taux d'expression desdites molécules d'expression protéiniques extraites, en se basant sur les produits de réaction quantifiés ;
(f) comparer lesdits taux d'expression déterminés avec les taux d'expression desdites molécules d'expression protéiniques extraites dans une référence ;
(g) déterminer une valeur de similarité entre un taux d'expression desdites molécules d'expression protéiniques extraites chez ledit individu et un taux d'expression des molécules d'expression protéiniques extraites chez un patient ne présentant pas de croissance cancéreuse ; et
(h) classifier ledit individu comme présentant une croissance cancéreuse si ladite valeur de similarité est inférieure à une première valeur seuil de similarité, et classifier ledit individu comme ne présentant pas de croissance cancéreuse si ladite valeur de similarité excède ladite première valeur seuil de similarité.

8. 5-fluorouracile (5-FU) destiné à être utilisé dans un procédé de traitement d'un individu qui est classifié comme présentant une croissance cancéreuse selon la revendication 7 et chez qui la présence d'une croissance cancéreuse a été confirmée par coloscopie.

9. 5-fluorouracile (5-FU) destiné à être utilisé selon la revendication 8, dans lequel le 5-FU est combiné avec la leucovorine.

10. 5-fluorouracile (5-FU) destiné à être utilisé selon la revendication 8 ou la revendication 9, dans lequel le procédé de traitement comprend le 5-FU, la leucovorine et l'oxaliplatine, ou le 5-FU, la leucovorine et l'irinotécan.

11. Capécitabine destinée à être utilisée dans un procédé de traitement d'un individu qui est classifié comme présentant une croissance cancéreuse selon la revendication 7 et chez qui la présence d'une croissance cancéreuse a été confirmée par coloscopie.

12. Capécitabine destinée à être utilisée selon la revendication 11, dans laquelle ladite capécitabine est combinée avec l'irinotécan et/ou l'oxaliplatine.
